(19) Europäisches Patentamt
European Patent Office
Office européen des brevets

(11) EP 1 751 126 B1

(12) # EUROPÄISCHE PATENTSCHRIFT

(45) Veröffentlichungstag und Bekanntmachung des Hinweises auf die Patenterteilung:
**01.09.2010 Patentblatt 2010/35**

(21) Anmeldenummer: **05716736.3**

(22) Anmeldetag: **18.02.2005**

(51) Int Cl.:
***C07D 305/12*** *(2006.01)* ***A61K 31/335*** *(2006.01)*
***A61P 3/00*** *(2006.01)*

(86) Internationale Anmeldenummer:
**PCT/EP2005/050719**

(87) Internationale Veröffentlichungsnummer:
**WO 2005/080366 (01.09.2005 Gazette 2005/35)**

(54) **ALKYLCARBAMAT-SUBSTITUIERTE BUTYROLACTONE ALS LIPASE-INHIBITOREN**

ALKYL CARBAMATE-SUBSTITUTED BUTYROLACTONES SERVING AS LIPASE INHIBITORS

BUTYROLACTONES SUBSTITUEES PAR CARBAMATE D'ALKYLE, UTILISEES COMME INHIBITEURS DE LIPASE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **23.02.2004 DE 102004009076**

(43) Veröffentlichungstag der Anmeldung:
**14.02.2007 Patentblatt 2007/07**

(73) Patentinhaber: **Abbott Products GmbH**
**30173 Hannover (DE)**

(72) Erfinder:
- **WALDECK, Harald**
  **30916 Isernhagen (DE)**
- **ANTEL, Jochen**
  **31848 Bad Münder (DE)**
- **EYTING, Sabine**
  **48159 Münster (DE)**
- **WURL, Michael**
  **30826 Garbsen (DE)**
- **GREGORY, Peter-Colin**
  **30559 Hannover (DE)**
- **WOLFF, Maike**
  **30171 Hannover (DE)**

(74) Vertreter: **Gosmann, Martin et al**
**Abbott Products GmbH**
**IP Department (PH-ZP)**
**Hans-Böckler-Allee 20**
**30173 Hannover (DE)**

(56) Entgegenhaltungen:
**EP-A- 0 444 482** **WO-A-01/32670**
**US-A- 4 931 463** **US-A1- 2001 012 852**

- **CARDANI ET AL.: "Titanium Tetrachloride-Mediated Enantioselective Synthesis of trans beta-Lactones" TETRAHEDRON, Bd. 48, Nr. 27, 1992, Seiten 5557-5564, XP002330603**
- **NELSON ET AL.: "Catalytic Asymmetric Acyl Halide-Aldehyde Cyclocondensation Reactions of Substituted Ketenes" J. AM. CHEM. SOC., Bd. 126, 18. Dezember 2003 (2003-12-18), Seiten 14-15, XP002330604**
- **YANG H W ET AL: "Studies of the tandem Mukaiyama aldol-lactonization (TMAL) reaction: A concise and highly diastereoselective route to beta-lactones applied to the total synthesis of the potent pancreatic lipase inhibitor, (-)-Panclicin D" TETRAHEDRON, ELSEVIER SCIENCE PUBLISHERS, AMSTERDAM, NL, Bd. 53, Nr. 48, 1. Dezember 1997 (1997-12-01), Seiten 16471-16488, XP004627154 ISSN: 0040-4020**
- **MEAD ET AL.: "A new approach to the preparation of 2-substituted tetrahydrofurans with alpha-syn selectivity" TETRAHEDRON LETTERS, Bd. 30, Nr. 49, 1989, Seiten 6829-6832, XP002330605**
- **CUNXIANG Z ET AL: "A beta-Lactone-Based Route to Cyclopentanes via Intramolecular Allylsilane Additions. An Unexpected Friedel-Crafts Alkylation" TETRAHEDRON LETTERS, ELSEVIER SCIENCE PUBLISHERS, AMSTERDAM, NL, Bd. 38, Nr. 37, 15. September 1997 (1997-09-15), Seiten 6537-6540, XP004089449 ISSN: 0040-4039**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 751 126 B1

## Beschreibung

Die vorliegende Erfindung betrifft durch eine unverzweigte Alkylcarbamat-Seitenkette substituierte β-Lactone (Oxetanone), welche geeignet sind für die Behandlung und/oder Prophylaxe von Obesitas sowie von damit einhergehenden Begleit- und/oder Folgeerkrankungen bei größeren Säugetieren und Menschen, insbesondere dem Metabolischen Syndrom und kardiovaskulären Erkrankungen. Ferner betrifft die Erfindung diese neuen Verbindungen enthaltende pharmazeutische Zubereitungen sowie Verfahren zur Herstellung dieser Verbindungen. Die erfindungsgemäßen Verbindungen wirken dabei als Inhibitoren der Lipase, insbesondere der Pankreaslipase.

Aus der EP 0 444 482 A2 sind bereits durch eine verzweigte Seitenkette substituierte Oxetanone mit die Pankreaslipase hemmender Wirkung bekannt.

Aus der EP 0 129 748 A1 sind bereits Hexadecansäure- und Hexadecadiensäurederivate bekannt, welche die Pankreaslipase hemmen und daher bei der Bekämpfung oder Verhütung von Obesitas und Hyperlipämien verwendet werden können.

Die Schrift WO 03/050154 A2 beschreibt unter anderem auch durch Seitenketten substituierte Oxetanone als Substrate für die katalytische Carbonylierung.

Silvia Cardani, Carlo De Toma, Cesare Gennari und Carlo Scolastico offenbaren in "Titanium Tetrachloride-Mediated Enantioselective Synthesis of trans β-Lactones", Tetrahedron, Bd. 48, Nr. 27, 1992, Seiten 5557-5564 spezielle trans β-Lactone auf Basis von Oxetan-2-onen.

Scott G. Nelson, Cheng Zhu und Xiaoqiang Shen offenbaren in "Catalytic Asymmetric Acyl Halide-Aldehyde Cyclocondensation Reactions of Substituted Ketens", J. Am. Chem. Soc., Bd. 126, 18.12.2003, Seiten 14 bis 15 spezielle trans β-Lactone auf Basis von Oxetan-2-onen mit Benzylsubstituenten als Alkoholschutzgruppe.

Hong Woon Yang, Cunxiang Zhao und Daniel Romo offenbaren in "Studies of the tandem Mukaiyama aldollactonization (TMAL) reaction: A consice and highly diastereoselective route to beta-lactones applied to the total synthesis of the potent pancreatic lipase inhibitor, (-)-Panclicin D" Tetradehdron, Bd. 53, Nr. 48, 01.12.1997, Seiten 16471-16488 spezielle β-Lactone auf Basis von Oxetan-2-onen.

Keith T. Mead und Hui-Li Yang offenbaren in "A new approach to the preparation of 2-substituted tetrahydrofurans with alpha-syn selectivity" Tetrahedron Letters, Bd. 30, Nr. 49, 1989, Seiten 6829-6832 ein spezielles trans β-Lactone auf Basis von Oxetan-2-onen mit Benzylsubstituenten als Alkoholschutzgruppe.

Cunxiang Zhao und Daniel Romo offenbaren in ""A beta-Lactone-based Route to Cyclopentanes via Intramolecular Allylsilane Additions. An unexpected Friedel-Crafts Alkylation" Tetrahedron Letters, Bd. 38, Nr. 37, 15.9.1997, Seiten 6537-6540 ein spezielles β-Lactone auf Basis von Oxetan-2-onen.

Aufgabe der vorliegenden Erfindung war es, neue Lipase-inhibitorisch wirksame Arzneimittel zur Behandlung und/oder Prophylaxe von Obesitas sowie deren Begleit- und/oder Folgeerkrankungen zur Verfügung zu stellen, welche gut wirksam sind und auf einfachem Wege erhalten werden können.

Es wurde nun gefunden, daß eine Gruppe von in der Alkylcarbamat-Seitenkette unverzweigten Alkylcarbamat-substituierten β-Lactonen als Inhibitoren der Lipase, insbesondere der Pankreaslipase, wirken können. Die erfindungsgemäßen Verbindungen sind somit in der Lage, die durch Pankreaslipase induzierte Fettverdauung bei Säugetieren, insbesondere Menschen, herabzusetzen, mit der Folge, daß dem Körper insgesamt weniger verwertbare Nahrungsfette zur Verfügung stehen. Die erfindungsgemäßen Verbindungen erscheinen daher geeignet zur Behandlung und/oder Prophylaxe von Obesitas und damit im Zusammenhang stehenden Erkrankungen.

Gegenstand der Erfindung sind Lipase-inhibitorisch wirksame Alkylcarbamat-substituierte β-Lactone der allgemeinen Formel I,

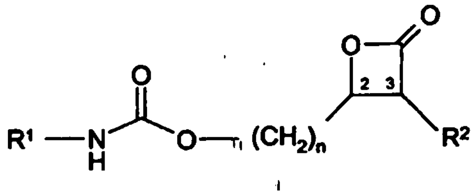

I

worin,

$R^1$ $C_{1-18}$-Alkyl, dessen Alkylketten-Kohlenstoffatome gegebenenfalls 1-2-fach durch Sauerstoff und/oder Schwefel ersetzt sind; Phenyl-$C_{0-18}$-alkyl, dessen Phenylgrup- pe gegebenenfalls 1-2-fach durch Halogen, Trifluormethyl, Nitro, $C_{1-8}$-Alkyl, $C_{1-8}$- Alkoxy, $C_{1-6}$-Alkylcarbonyl, $C_{1-6}$-Alkoxycarbonyl, Phenyl, Benzyl und/oder Phenoxy substituiert ist und dessen Alkylketten-Kohlenstoffatome gegebenenfalls 1-2-fach durch Sauerstoff und/oder Schwefel ersetzt sind; $C_{3-7}$-Cycloalkyl-$C_{0-18}$-Alkyl, dessen Alkylketten-Kohlenstoffatome gegebenenfalls 1-2-fach durch

Sauerstoff und/oder Schwefel ersetzt sind, oder Benzoyl bedeutet;

$R^2$ $C_{1-12}$-Alkyl, dessen Alkylketten-Kohlenstoffatome gegebenenfalls 1-2-fach durch Sauerstoff und/oder Schwefel ersetzt sind; Phenyl-$C_{1-18}$-alkyl, dessen Phenylgrup- pe gegebenenfalls 1-2-fach durch Halogen, $C_{1-4}$-Alkyl, $C_{1-4}$-Alkoxy, Benzyl und/oder Phenoxy substituiert ist und dessen Alkylketten-Kohlenstoffatome gegebenenfalls 1-2-fach durch Sauerstoff und/oder Schwefel ersetzt sind; oder $C_{3-7}$-Cycloalkyl-$C_{0-18}$-Alkyl, dessen Alkylketten-Kohlenstoffatome gegebenenfalls 1-2-fach durch Sau- erstoff und/oder Schwefel ersetzt sind, bedeutet, und

n eine ganze Zahl von 2 bis 8 bedeutet.

Weiterhin sind Gegenstand der Erfindung die Verbindungen der Formel I enthaltenden Arzneimittel. Ferner sind Gegenstand der Erfindung ein Verfahren zur Herstellung der Verbindungen der Formel I und Zwischenprodukte dieses Verfahrens.

Sofern in den Verbindungen der Formel I oder in anderen im Rahmen der vorliegenden Erfindung beschriebenen Verbindungen Substituenten Alkyl bedeuten oder enthalten, kann dieses jeweils geradkettig oder verzweigt sein. Sofern Substituenten in Verbindungen der Formel I für Halogen stehen, kommen Fluor, Chlor oder Brom in Frage. Chlor ist bevorzugt.

$R^1$ bedeutet vorzugsweise Phenyl-$C_{0-2}$-alkyl, dessen Phenylgruppe gegebenenfalls wie vorstehend angegeben substituiert ist. Bevorzugte Substituenten sind $C_{1-4}$-AIW, $C_{1-4}$-Alkoxy und Phenoxy. Insbesondere kann $R^1$ für 4-Phenoxyphenyl, Benzyl, Phenylethyl oder 4-Ethoxybenzyl stehen. 4-Phenoxyphenyl ist besonders bevorzugt.

$R^2$ bedeutet vorzugsweise $C_{2-6}$-Alkyl, dessen Alkylketten-Kohlenstoffatome gegebenenfalls 1-2-fach durch Sauerstoff und/oder Schwefel ersetzt sind, insbesondere Hexyl. Sofern $R^2$ gegebenenfalls in der Phenylgruppe substituiertes Phenyl-$C_{1-18}$-alkyl bedeutet, ist Phenyl-$C_{2-4}$-alkyl bevorzugt.
n bedeutet vorzugsweise eine ganze Zahl von 2 bis 5.

Verbindungen der Formel I, worin die Substituenten des Kohlenstoffs in 2-Stellung des Lactonrings und die Substituenten des Kohlenstoffs in 3-Stellung des Lactonrings die trans-Stellung zueinander einnehmen, sind bevorzugt.

Besonders bevorzugte Verbindungen der Formel I sind ausgewählt aus der Gruppe bestehend aus 5-(3-Hexyl-4-oxo-cxetan-2-yl)-pentyl-(phenoxyphenyl)-carbamat und 4-(3-Hexyl-4-oxo-oxetan-2-yl)-butyl-(phenoxyphenyl)-carbamat, welche jeweils besonders bevorzugt sind in ihrer 2S,3S-trans-Form.

Es sind zahlreiche Verfahren zur Herstellung von β-Lactonen (Oxetanen) bekannt (vgl. z.B: A. Pommier, J.-M. Pons, Synthesis 5 (1993) 441 - 459). Erfindungsgemäß werden die neuen Verbindungen der Formel I erhalten, indem man eine Verbindung der allgemeinen Formel II,

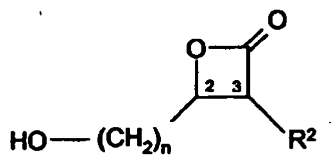

II

worin $R^2$ und n obige Bedeutungen besitzen, mit einer Verbindung der allgemeinen Formel III,

$R^1$-N=C=O III

worin $R^1$ obige Bedeutung besitzt, umsetzt.

Die Umsetzung des Alkohol-Derivates der Formel II mit einer Isocyanat-Verbindung der Formel III kann auf an sich bekannte Weise durchgeführt werden, beispielsweise in einem unter den Reaktionsbedingungen inerten Lösungsmittel wie einem dipolaraprotischen Lösungsmittel, insbesondere einem Niederalkylhalogenid, vorzugsweise Dichlormethan. Übliche Reaktionstemperaturen liegen zwischen etwa -25 °C und etwa 50 °C. Meist wird die Reaktionstemperatur während des Zusammenfügens der Reaktionspartner zwischen etwa -25 °C und 10 °C, vorzugsweise bei 0 °C, gehalten und nach vollendetem Zusammenfügen auf etwa 15 °C bis 50 °C, vorzugsweise auf Raumtemperatur (= RT) erhöht. In einigen Fällen ist es günstig, nach einer gewissen Reaktionszeit, beispielsweise nach 2 Stunden, dem Reaktionsgemisch eine nicht-nucleophile Base, beispielsweise eine organische Stickstoffbase wie insbesondere Diisopropylethylamin (= "Hünig-Base"), hinzuzufügen und anschließend zur Vervollständigung der Reaktion eine gewisse Zeit, beispielweise erneut 2 Stunden lang, weiterreagieren zu lassen. Verbindungen der Formel I können anschließend auf an sich bekannte Weise aus dem Reaktionsgemisch isoliert und nötigenfalls gereinigt werden.

Verbindungen der Formel II können erhalten werden, indem man aus an der Alkoholfunktion geschützten Lacton-Verbindungen der allgemeinen Formel IVa,

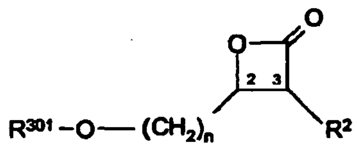

IVa

worin $R^2$ und n obige Bedeutungen besitzen und $R^{301}$ für eine Alkoholschutzgruppe steht, auf an sich bekannte Weise die Alkoholschutzgruppe abspaltet. Geeignete Schutzgruppen für Alkoholfunktionen sowie Methoden zu deren Einführung und Abspaltung sind beispielsweise bekannt aus McOmie, "Protective Groups in Organic Chemistry", Plenum Press, und Greene, Wuts, "Protective Groups in Organic Synthesis", Wiley Interscience Publication, in der jeweils jüngsten Auflage. Besonders geeignete Alkoholschutzgruppen $R^{301}$ sind ausgewählt aus der Grupppe bestehend aus Benzyl, Tetrahydropyranyl, Methoxymethyl (= MOM), Methoxyethoxymethyl (= MEM) und den Silyl-Alkoholschutzgruppen, insbesondere tertiär-Butyldimethylsilyl und Triethylsilyl.

Verbindungen der Formel II sind neue Verbindungen, welche sich in vorteilhafter Weise als Zwischenprodukte zur Herstellung neuer pharmakologisch aktiver Wirkstoffe, beispielsweise zur Herstellung der Verbindungen der Formel I eignen.

Verbindungen der Formel III sind an sich bekannt oder können auf an sich bekannte Weise aus bekannten Verbindungen hergestellt werden.

Verbindungen der Forme IVa eignen sich in vorteilhafter Weise als Zwischenprodukte zur Herstellung neuer pharmakologisch aktiver Wirkstoffe, beispielsweise zur Herstellung der Verbindungen der Formel I. Die neue Verbindung 3-Hexyl-4-{5[(tetrahydro-2*H*-pyran-2-yl)oxy]pentyl}oxetan-2-on zeigt ebenfalls bereits eine die Pankreaslipase inhibierende Wirkung und erscheint daher ebenfalls angezeigt zur Behandlung und/oder Prophylaxe von Obesitas sowie deren Begleit- und/oder Folgeerkrankungen. Aus Verbindungen der Formel IV können die Alkoholschutzgruppen $R^{301}$ auf an sich bekannte Weise wieder abgespalten werden, um die freien Alkoholfunktionen zu erhalten. Auf diese Weise werden Verbindungen der allgemeinen Formel IV zugänglich,

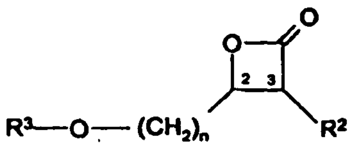

IV

worin $R^2$ und n obige Bedeutungen besitzen und $R^3$ Wasserstoff oder eine Alkoholschutzgruppe bedeutet.

Verbindungen der Formel IVa können erhalten werden, indem man in einer ersten Variante eine β-Hydroxycarbonsäure der allgemeinen Formel V,

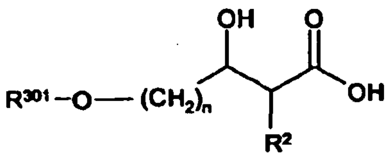

V

worin $R^2$, $R^{301}$ und n obige Bedeutungen besitzen, auf an sich bekannte Weise zu einem β-Lacton cyclisiert. Die Cyclisierung kann beispielsweise durch Umsetzung der Verbindung der Formel IVa mit einem Reagenz durchgeführt werden, welches zusammen mit einer Carbonsäurefunktion eine leicht abspaltbare Fluchtgruppe erzeugt, wie mit einem Sulfonsäurederivat, beispielsweise mit Toluolsulfonsäurechlorid. Ebenso ist es auch möglich, in einer Verbindung der Formel IVa auf an sich bekannte Weise die freie Alkoholfunktion entsprechend zu aktivieren, um sie dem nucleophilen Angriff der gegebenenfalls aktivierten Carbonsäurefunktion, beispielsweise des Carboxylats, zugänglich zu machen.

Verbindungen der Formel V können in einer ersten Variante hergestellt werden, indem man einen Aldehyd der allgemeinen Formel VI,

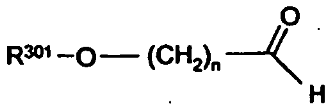

VI

worin $R^{301}$ und n obige Bedeutungen besitzen, auf an sich bekannte Weise mit einem Carbonsäurederivat der allgemeinen Formel VII,

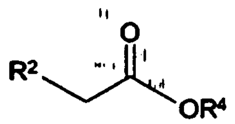

VII

worin $R^2$ obige Bedeutung besitzt und $R^4$ Wasserstoff oder $C_{1-4}$-Alkyl bedeutet, umsetzt. Zur Durchführung der Reaktion kann eine Verbindung der Formel VII zuerst auf an sich bekannte Weise mit einer starken nicht-nucleophilen Base wie Lithiumdiisopropylamid (= LDA) - gegebenenfalls zweifach - deprotoniert werden und das entstandene Carbanion kann anschließend mit der Verbindung der Formel VI umgesetzt werden. Die nach dieser vorgenannten Variante hergestellten Verbindungen der Formel V weisen in der Regel keine definierte Stereochemie an den die Substituenten "$R^2$" und "β-Hydroxy" tragenden Kohlenstoffatomen auf, und liegen daher als "syn/anti-Gemische" vor.

Aldehyde der Formel VI können hergestellt werden durch an sich bekannte selektive Oxidation von primären Alkoholen der allgemeinen Formel VIII,

$R^{301}$-O- $(CH_2)_n$ -OH VIII

worin $R^{301}$ und n obige Bedeutungen besitzen. Die selektive Oxidation kann beispielsweise als sogenannte "Swern-Oxidation" durchgeführt werden, wobei als Oxidationsmittel Dimethylsulfoxid (= DMSO) in Anwesenheit eines Elektrophils, beispielsweise Oxalylchlorid, eingesetzt wird. Ferner kann eine selektive Oxidation auch mit Pyridiniumchlorochromat (= PCC) als Oxidationsmittel durchgeführt werden. Verbindungen der Formel VIII sind an sich bekannt oder können auf an sich bekannte Weise hergestellt werden, insbesondere durch an sich bekannte Einführung geeigneter Alkoholschutzgruppen in die entsprechenden zugrundeliegenden terminalen Diole.

Verbindungen der Formel VII sind an sich bekannt, oder können auf an sich bekannte Weise aus bekannten Verbindungen hergestellt werden.

Verbindungen der Formel V können in einer zweiten Variante auch hergestellt werden, indem man eine Verbindung der allgemeinen Formel IX,

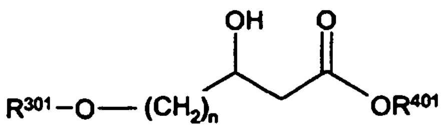

IX

worin $R^{301}$ und n obige Bedeutungen besitzen und $R^{401}$ $C_{1-4}$-Alkyl, insbesondere Methyl, bedeutet, auf an sich bekannte Weise zuerst mit einer starken nicht-nucleophilen Base wie einer Lithium-Niederalkylverbindung, vorzugsweise LDA, zweifach deprotoniert, und anschließend das deprotonierte Zwischenprodukt mit einer Verbindung der allgemeinen Formel X,

$R^2$-X X

worin $R^2$ obige Bedeutung besitzt und X für eine abspaltbare Fluchtgruppe, beispielsweise Halogen, insbesondere lod, Brom oder Chlor, vorzugsweise lod, steht, umsetzt, und die Estergruppe $R^{401}$ nachfolgend auf an sich bekannte Weise abspaltet. Die Deprotonierung sowie die anschließende Umsetzung mit einer Verbindung der Formel X kann in einem unter den Reaktionsbedingungen inerten organischen Lösungsmittel wie einem cyclischen oder offenkettigen Diniederalkylether, insbesondere Tetrahydrofuran (= THF), durchgeführt werden. In einer bevorzugten Variante können Verbindungen der Formel V zumindest in Diastereomeren-angereicherter Form, vorzugsweise diastereoselektiv, erhalten werden, wenn man dem Reaktionsgemisch vor Zugabe der Verbindung der Formel X einen geeigneten Komplexbildner

zugibt. Als Komplexbildner eignen sich beispielsweise Tris-(dimethylamino)-phosphin (= HMPT), 1,3-Dimethyl-3,4,5,6-tetrahydro-2(1H)-pyrimidinon (= DMPH) oder 1,3-Dimethylimidazolidin-2-on (= DMEU). DMPH ist bevorzugt. Die Verbindungen der Formel V werden unter diesen Bedingungen vorzugsweise in der "anti"-Konfiguration gebildet. Die vorgenannte Abspaltung der Estergruppe $R^{401}$ kann beispielsweise durch Verseifung mit einem Alkalihydroxid wie Lithiumhydroxid und in einem polar-protischen Lösungsmittel wie Ethanol durchgeführt werden.

Verbindungen der Formel IX können in einer ersten Variante hergestellt werden, indem man auf an sich bekannte Weise zuerst eine Verbindung der allgemeinen Formel XI,

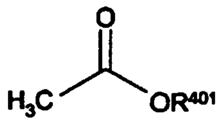

XI

worin $R^{401}$ obige Bedeutung besitzt, mit einer starken, nicht-nucleophilen Base wie einer Lithium-Niederalkylverbindung, vorzugsweise LDA, deprotoniert, und anschließend das deprotonierte Zwischenprodukt mit einer Verbindung der Formel VI umsetzt. Die Deprotonierung sowie die anschließende Umsetzung mit einer Verbindung der Formel VI kann in einem unter den Reaktionsbedingungen inerten organischen Lösungsmittel wie einem Diniederalkylether, insbesondere THF, durchgeführt werden. Üblicherweise wird bei niedrigen Temperaturen zwischen etwa -80 °C und -50 °C gearbeitet.

Verbindungen der Formel XI sind bekannt oder können auf an sich bekannte Weise aus bekannten Verbindungen hergestellt werden.

Vebindungen der Formel IX können in einer zweiten Variante in zumindest Enantiomeren-angereicherter Form, vorzugsweise enantiomerenrein, hergestellt werden, indem man eine racemische Ausgangsverbindung der Formel IX auf an sich bekannte Weise selektiv oxidiert zu einem β-Ketoester der allgemeinen Formel XII,

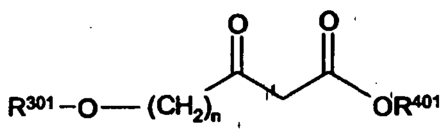

XII

worin $R^{301}$, $R^{401}$ und n obige Bedeutungen besitzen, und die entstandene Ketoester-Verbindung anschließend selektiv und in Anwesenheit eines geeigneten chiralen Katalysators zu einer zumindest Enantiomeren-angereicherten Verbindung der Formel IX reduziert. Die Oxidation einer Ausgangsverbindung der Formel IX kann in einem unter den Reaktionsbedingungen inerten organischen Lösungsmitttel wie einem Niederalkylhalogenid, insbesondere Dichlormethan, durchgeführt werden. Als selektives Oxidationsmittel eignet sich beispielsweise PCC. Die enantioselektive Reduktion eines β-Ketoesters der allgemeinen Formel XII kann unter Luftausschluß in einem unter den Reaktionsbedingungen inerten Lösungsmittel wie Dimethylformamid (= DMF) oder Methanol oder in einem Gemisch der vorgenannten Lösungsmittel durchgeführt werden. Die Hydrierung kann bei einem Wasserstoffdruck von etwa 2 - 6 bar, vorzugsweise bei etwa 4,0 - 4,5 bar, durchgeführt werden. Geeignete Reaktionstemperaturen liegen zwischen etwa 50 °C und etwa 90 °C, vorzugsweise zwischen etwa 60 °C und 80 °C. Als chirale Hydrierungskatalysatoren eignen sich insbesondere an sich bekannte Komplexe von Ruthenium-II mit (S)-(-)-2,2'-Bis-(diphenylphosphino)-1,1'-binaphthyl (= (S)-(-)-BINAP) oder von Ruthenium-II mit (R)-(+)2,2'-Bis-(diphenylphosphino)-1,1'-binaphthyl (= (R)-(+)-BINAP). In Abhängigkeit von der Stereochemie, welche in dem chiralen Hydrierungskatalysator vorliegt, kann eine jeweils an (S)-Isomer oder an (R)-Isomer zumindest angereicherte Verbindung der Formel IX erhalten werden. Sofern (S)-(-)-BINAP als Ligand des chiralen Katalysators eingesetzt wird, werden in der Regel Verbindungen der Formel IX erhalten, worin der die neu erzeugte Alkoholfunktion tragende Kohlenstoff die "S"-Konfiguration aufweist.

Verbindungen der Formel IVa können auch erhalten werden, indem man in einer zweiten Variante auf an sich bekannte Weise eine Silyl-Ketenacetal-Verbindung der allgemeinen Formel XIII,

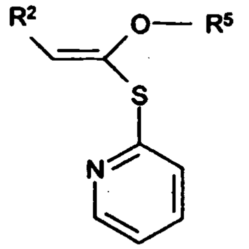

XIII

worin $R^2$ obige Bedeutung besitzt und $R^5$ eine Silylgruppe, insbesondere Triethylsilyl, bedeutet, im Sinne einer Tandem-Mukaiyama-Aldol-Lactonisierung (vgl. z.B. H.W. Yang et al., Tetrahedron 53 No. 48 (1997) 16471-16488) mit einem Aldehyd der Formel VI umsetzt. Die Reaktion kann beispielsweise unter Schutzgasatmosphäre in einem organischen Lösungsmittel wie einem Niederalkylhalogenid, insbesondere Dichlormethan, und in Anwesenheit einer Lewis-Säure, insbesondere einem Zn-II-Salz wie Zn-II-chlorid durchgeführt werden. In dieser Reaktionsvariante werden in der Regel Gemische von Verbindungen der Formel IVa erhalten, deren Substituenten "$R^2$" und "$R^{301}$-$(CH_2)_n$-" überwiegend in der "trans"-Konfiguration zueinander vorliegen.

Verbindungen der Formel XIII können hergestellt werden, indem man eine Verbindung der allgemeinen Formel XIV,

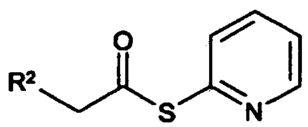

XIV

worin $R^2$ obige Bedeutung besitzt, auf an sich bekannte Weise zuerst mit einer starken, nicht-nucleophilen Base deprotoniert und anschließend das deprotonierte Zwischenprodukt mit einem zur Einführung einer Silylschutzgruppe geeigneten Reagenz umsetzt. Die Reaktion kann in einem organischen Lösungsmittel wie DMF oder THF oder in einem Gemisch dieser Lösungsmittel durchgeführt werden. Als starke, nicht-nucleophilen Base eignet sich beispielsweise Lithiumhexamethyldisilazid. Vorzugsweise wird die Reaktion bei Temperaturen zwischen etwa - 90 °C und -60 °C durchgeführt. Als zur Einführung einer Silylschutzgruppe geeignetes Reagenz kann beispielsweise Triethylsilylchlorid verwendet werden.

Verbindungen der Formel XIV können erhalten werden, indem man auf an sich bekannte Weise eine Verbindung der allgemeinen Formel XV,

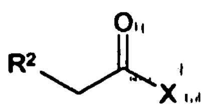

XV

worin $R^2$ und X obige Bedeutungen besitzen, mit 2-Mercaptopyridin umsetzt.

Die Verbindungen der Formel I enthalten zwei chirale Kohlenstoffatome, nämlich den den Substituenten "$R^1$-N-C(O)-$(CH_2)_n$-" tragenden Kohlenstoff in 2-Stellung des Lactonringes und den den Substituenten "-$R^2$" tragenden Kohlenstoff in 3-Stellung des Lactonringes. Die Verbindungen können somit in insgesamt vier stereoisomeren Formen vorliegen. Die vorliegende Erfindung umfaßt sowohl die Gemische von Stereoisomeren bzw. Enantiomeren, wie auch die isomerenreinen Verbindungen der Formel I. Bevorzugt sind isomerenreine Verbindungen der Formel I, insbesondere solche, worin die vorgenannten Substituenten der Kohlenstoffatome in 2- und in 3-Stellung des Lactonringes in der "trans"-Konfiguration vorliegen. Üblicherweise sind Verbindungen der Formel I besonders bevorzugt, worin der den Substituenten "$R^1$-N-C(O)-$(CH_2)_n$-" tragende Kohlenstoff in 2-Stellung des Lactonringes und der den Substituenten "-$R^2$" tragende Kohlenstoff in 3-Stellung des Lactonringes beide die "S"-Konfiguration aufweisen.

Bei den vorstehend beschriebenen Umsetzungen werden die gegebenenfalls vorgebildeten chiralen Zentren in den Ausgangsverbindungen der Formel V in den nachfolgenden Umsetzungen zu Verbindungen der Formel I nicht mehr verändert, so daß je nach Art der Ausgangsverbindungen schließlich isomerenreine Verbindungen der Formel I oder Isomerengemische erhalten werden können. Zur Herstellung von stereochemisch einheitlichen Verbindungen der Formel I werden zweckmäßigerweise stereochemisch einheitliche Verbindungen der Formel V eingesetzt. Werden

Gemische von Isomeren der Formel I erhalten, so können diese gewünschtenfalls auf an sich bekannte Weise getrennt werden, beispielsweise durch Chromatographie, insbesondere durch HPLC-Trennung an gegebenenfalls chiralen Trennmaterialien.

Die erfindungsgemäßen Verbindungen der Formel I eignen sich zur Hemmung der Lipase, insbesondere zur gegebenenfalls selektiven Hemmung der Pankreaslipase von größeren Säugetieren, insbesondere Menschen. Verbindungen mit lipasehemmenden Eigenschaften sind in der Lage, sofern sie vorzugsweise gemeinsam mit fetthaltiger Nahrung dem Verdauungstrakt zugeführt werden, den Anteil der vom Körper tatsächlich verdauten Nahrungsfette an den insgesamt aufgenommenen Nahrungsfetten herabzusetzen. Auf diese Weise kann die Fettresorption bei Säugetieren, insbesondere Menschen, herabgesetzt werden. Die erfindungsgemäße Gruppe von Verbindungen erscheint somit geeignet zur Behandlung und/oder Prophylaxe von Obesitas sowie von damit einhergehenden Begleiterkrankungen und/oder Folgeerkrankungen. Zu den begleitenden Erkrankungen der Obesitas oder deren Folgeerkrankurigen, welche jeweils mit den erfindungsgemäßen Verbindungen therapierbar sein können, zählen insbesondere das Metabolische Syndrom und kardiovaskuläre Erkrankungen. Unter dem Begriff "Metabolisches Syndrom" wird üblicherweise ein Komplex von Krankheitsbildern zusammengefaßt, welcher hauptsächlich Bluthochdruck (= Hypertonie), insbesondere arteriellen Bluthochdruck, Insulinresistenz, insbesondere Diabetes mellitus Typ II, Dyslipoproteinämie, insbesondere als Hypertriglyceridämie - einhergehend mit erniedrigtem HDL-Cholesterin auftretende Dyslipoproteinämie - sowie Hyperurikämie, welche zur Gicht führen kann, umfaßt. Unter dem Begriff "kardiovaskuläre Erkrankungen" werden im Zusammenhang mit Obesitas üblicherweise die koronare Herzkrankheit, welche zur Herzinsuffizienz führen kann, cerebrovasculäre Erkrankungen, welche beispielsweise mit einem erhöhten Schlaganfallrisiko einhergegen können, und die periphere arterielle Verschlußkrankheit verstanden. Weitere Begleit- und/oder Folgeerkrankungen der Obesitas können Gallenblasenerkrankungen wie Gallensteinbildung, das Schlafapnoe-Syndrom, orthopädische Komplikationen wie Osteoarthritis sowie psycho-soziale Störungen sein.

Beschreibung der pharmakologischen Testmethoden

1. p-Nitrophenylpalmitat-Test

Die Lipase-hemmenden Eigenschaften der Verbindungen der Formel I lassen sich z. B. durch einen in vitro-Aktivitätstest belegen. In diesem Test wird die Hemmung der lipolytischen Wirkung von Schweinepankreaslipase gegenüber dem Testsubstrat p-Nitrophenylpalmitat (= pNpp) unter dem Einfluß der Testsubstanzen der Formel I bestimmt. Dabei wird die Änderung der relativen Extinktion der Untersuchungslösungen gemessen, welche durch die lipolytische Freisetzung von p-Nitrophenol aus pNpp verursacht wird.

Es wurden die nachfolgend angegebenen Reagenzien hergestellt:

A. Substratlösung

Zur Herstellung einer "Lösung A" wurden 45 mg pNpp in 15 ml Isopropanol durch Beschallen mit Ultraschall gelöst. Zur Herstellung einer "Lösung B" wurden 558 mg Na-Deoxycholat-Trockensubstanz und 67,5 mg Gummi arabisch in 135 ml 0,05 M Natriumphosphatpuffer (pH = 8,0) gelöst. In 135 ml eiskalte "Lösung B" wurden 15 ml "Lösung A" in 8 Schritten zu 1,25 ml mit einem Eppendorf-Handdispenser unter Rühren bei 400 Upm mit möglichst hoher Geschwindigkeit eingespritzt. Die Anfangsextinktion E1 bei Bestimmung des Leerwertes war jeweils kleiner als 0,250 E.

B. Natriumchloridlösung 1 % (m/V)

10 g Natriumchlorid (NaCl, p.a.) wurden ih 1000 ml Reinstwasser gelöst.

C. Lipase-Standardlösung (50 FIP-E/ml)

120,8 mg Lipase-Standard LS7 nach den Regeln der "Fédération Internationale Pharmaceutique" (= FIP) (Schweinepankreaslipase, 36.700 FIP-E/g) wurden in 50 ml eiskalter Natriumchloridlösung (B) gelöst und durch einen 0,2 μm-Spritzenfilter filtriert. 20 ml des Filtrats wurden mit 8 ml Natriumchloridlösung (B) verdünnt (entsprechend 50 FIP-E/ml).

D. Lipase-Kalibrierlösungen (10 - 20 - 30 - 40 FIP-E/ml)

Jeweils 2 - 4 - 6 - 8 ml Lipase-Standardlösung (C) wurden mit Natriumchloridlösung (B) auf 10 ml aufgefüllt und im Eisbad aufbewahrt.

E. Lipase-Stammlösung (40 FIP-E/ml)

8 ml Lipase-Standardlösung wurden mit Natriumchloridlösung (B) auf 10 ml aufgefüllt und im Eisbad aufbewahrt.

F. Inhibitorlösungen

Die Lipase-inhibitorisch wirksamen Verbindungen der Formel I wurden in DMSO gelöst. Aus dieser Stammlösung wurde eine Verdünnungsreihe von 100 - 0,1 nmol/ml erstellt. 100 μl dieser Verdünnungen wurden im Test eingesetzt. Die Verdünnungen wurden so angepaßt, daß die maximale Restaktivität der Lipase > 90 % betrug.

G. Durchführung

Die Messung erfolgte bei 37 °C, die Extinktionen wurden bei einer Wellenlänge von 405 nm gemessen. Der photometrische Meßplatz umfaßt ein Photometer und einen Schüttler (Fa. Eppendorf). Das Photometer wurde mit Wasser auf 0 E abgeglichen. Zum Anfang und zum Ende der Untersuchungsreihe wurden je acht Leerwerte und je eine Lipasekalibrierreihe gemessen. Für die Leerwerte wurde jeweils 1 ml Substratlösung mit 100 μl DMSO gemischt, 5 Sekunden lang geschüttelt und 5 Minuten lang temperiert. Dann wurden 100 μl Natriumchloridlösung (B) dazu pipettiert und es wurde weitere 5 Sekunden lang geschüttelt. Nach 2 Minuten wurde 4 Minuten lang die Extinktionszunahme erfasst. Der Extinktionsstartwert E1 bei der Ermittlung der Leerwerte war jeweils kleiner als 0,250 E.

Für die Kalibrierung wurde je 1 ml Substratlösung in acht Küvetten vorgelegt, mit 100 μl DMSO gemischt, 5 Sekunden lang geschüttelt und 5 Minuten lang temperiert. Die Reaktion wurde mit 100 μl Kalibrierlösung gestartet. Nach 2 Minuten erfolgte die Erfassung der Kinetik über 4 Minuten. Jede Konzentration wurde doppelt vermessen.

Für die Messung der Inhibitorlösungen wurde je 1 ml Substratlösung in acht Küvetten vorgelegt, mit 100 μl Inhibitorlösung gemischt, 5 Sekunden lang geschüttelt und dann 5 Minuten lang temperiert. Die Reaktion wurde mit 100 μl Lipase-Stammlösung gestartet. Nach 2 Minuten erfolgte die Erfassung der Kinetik über 4 Minuten. Die Inhibitorlösungen und die Lipase-Standardlösung wurden jeweils unmittelbar vor Beginn der Untersuchungsreihe hergestellt.

Zur Berechnung der Ergebnisse wurde aus den Kalibrierdaten und dem Leerwert mittels linearer Regression die Geradengleichung, der Achsenabschnitt und das Bestimmtheitsmaß nach folgender Gleichung bestimmt:

$$y = mx + b$$

x = Lipaseeinheiten FIP-E; y = Extinktion in Δ E/min;m = Steigung in (Δ E/min)/E; b = Achsenabschnitt

Aus diesen Daten wurde die unbekannte Aktivität x einer Lipaseinhibitortösung nach folgender Formel berechnet:

$$x = (y - b) / m$$

Für die enzymatische Restaktivität [in %] wurde folgende Formel verwendet:

$$\% = x * 100 / 4 \text{ FIP-E}$$

Als Maß für die inhibitorische Wirksamkeit der Substanzen der Formel I wurde deren $IC_{50}$-Wert bestimmt. Hierzu wurden die für die einzelnen Verbindungen der Formel I gemessenen Inhibitionswerte in Prozent in Konzentrationen umgerechnet und mit Hilfe des PRISM-3-Rechenprogrammes nach folgendem Algorithmus in $IC_{50}$-Werte umgerechnet: nichtlinearer Kurvenfit, sigmoidaler Kurvenverlauf mit variabler Steigung; 100 % Inhibition als Maximalwert, 0 % Inhibition als Minimalwert wurden als Konstanten hinzugefügt. Der $IC_{50}$-Wert einer enzyminhibierend wirksamen Testsubstanz ist diejenige Konzentration der Testsubstanz, bei der das Enzym unter sonst gleichen Testbedingungen noch 50 % Restaktivität besitzt. In dem vorstehend angegebenen Pankreaslipase-Aktivitätstest zeigten die in der nachfolgend angegebenen Tabelle 1 aufgeführten Testsubstanzen der Formel I die nachfolgend angegebenen $IC_{50}$-Werte. Die angegebenen Beispielsnummem beziehen sich auf die nachfolgend angegebenen Herstellungsbeispiele.

Tabelle 1: Schweinepankreaslipase-inhibierende Wirkung der Testsubstanzen in vitro gegenüber pNpp

| Beispiel Nr. | $IC_{50}$ [nM] |
|---|---|
| 1 | 106 |
| 2 | 47 |
| 3 | 88 |
| 6 | 113 |
| 7 | 107 |
| 8 | 187 |
| 9 | 67 |
| 10 | 177 |
| 11 | 28 |
| 13 | 319 |

2. Olivenöl-Test

Die Lipase-hiemmenden Eigenschaften der Verbindungen der Formel I lassen sich auch durch einen weiteren in vitro-Aktivitätstest in belegen. In einer heterogenen Reaktion wird die Hemmung der lipolytischen Wirkung von Schweinepankreaslipase unter dem Einfluß der Testsubstanzen der Formel I gegenüber den in dem Testsubstrat Olivenöl enthaltenen Triglyceriden bestimmt. Die durch die Lipase freigesetzten Fettsäuren wurden mit Natronlauge bei pH 9.0 titriert.

Es wurden die nachfolgend angegebenen Reagenzien hergestellt:

A. Gummi arabicum-Lösung 10 % (m/V)

200 g Gummi arabisch (Acaciae gummi entsprechend den Vorschriften des Deutschen Arzneibuches (= DAB) / der European Pharmacopeia (= Ph.Eur.), wurden in 2000 ml Reinstwasser unter Rühren und nötigenfalls Zentrifugieren gelöst. Die Lösung wurde bei -20 °C in Plastikgefäßen mit 250 ml Inhalt gelagert. Die täglich benötigte Menge wurde bei Bedarf aufgetaut.

B. Natriumtaurocholat-Lösung 8 % (m/V)

8 g Natriumtaurocholat (FIP) wurden in 100 ml Reinstwasser gelöst.

C. Puffer-Lösung

60,6 mg Tris(hydroxymethyl)aminomethan p.a. (Merck, no. 8382) und 234 mg Natriumchlorid (p.a.) wurden in 100 ml Reinstwasser gelöst.

D. 0,1 N Natronlauge

E. Olivenöl-Emulsion

40 ml Olivenöl (DAB, Raumtemperatur), 330 ml Gummi arabisch-Lösung (A.) und 30 g Eis (aus Reinstwasser) wurden in einem geeigneten Mixer 15 Minuten lang emulgiert. Die Emulsion wurde für den jeweiligen Verwendungstag frisch hergestellt.

F. Reagenzienmischung

100 ml Natriumtaurocholat-Lösung (B.), 400 ml Puffer-Lösung (C.) und 450 ml Reinstwasser wurden unter Rühren gemischt. Die Mischung wurde täglich frisch angesetzt. Für jede Messung wurden 19 ml benötigt.

G. Lipase-Lösungsmittel

10,0 g Natriumchlorid (p.a.), 6,06 g Tris(hydroxymethyl)aminomethan (p.a. Merck, no. 8382) und 4,9 g Maleinsäureanhydrid (p.a.) wurden in 900 ml Reinstwasser gelöst. Mit 4 N Natronlauge (p.a.) wurde ein pH-Wert von 7,0 eingestellt. Mit Reinstwasser wurde die Lösung auf 1000,0 ml aufgefüllt. Lagerung bei 5°C+/-3°C bis zu 3 Monaten.

H. Inhibitor-Stammlösungen

Aus den Inhibitor-Verbindungen der Formel I wurden jeweils 5 ml einer 0,02 mM Stammlösung in DMSO hergestellt. Aus der Stammlösung wurden dann Verdünnungen in einem Konzentrationsbereich von 20000 - 0,00002 nmol/Test erstellt: Aliquots dieser Lösungen wurden im Test eingesetzt. Die Verdünnungen und Volumina im Test wurden so angepaßt, daß die maximale Restaktivität der Lipase > 90 % betrug.

I. Referenzsuspension ( Lipase-Referenz-Standard)

Ca. 2500 FIP/Ph.Eur.-E Lipase-Referenz-Standard wurden in einem Becherglas genau abgewogen, mit etwas Seesand und einigen Tropfen eiskaltem Lipase-Lösungsmittel (G.) mit einem Glasstab zu einem Brei angerieben und mit 200 ml eiskaltem Lipase-Lösungsmittel 15-30 Min im Eisbad gerührt. Von dieser Lösung wurde 1 ml im Test eingesetzt.

J. Reaktionslösung

In einem thermostatisierten Reaktionsgefäß wurden 10 ml Olivenöl-Emulsion (E.) und 19 ml Reagenzienmischung (F.) bei 37,0°C +/- 0,1°C temperiert. Der pH-Wert wurde mit 0,1 mol/l Natronlauge (D.) auf pH 9,0 vortitriert.

K. Lipase-Inhibitor-Lösung

1 ml Lipase-Referenzsuspension wurde mit der jeweiligen Menge Inhibitorlösung 10 Minuten lang im Eisbad inkubiert. Nach der Inkubationszeit wurde die Lipase-Inhibitorlösung zur Reaktionslösung pipettiert. Dadurch wurde die Reaktion gestartet. Die freigesetzte Säure wurde bei 37,0°C +/- 0,5°C unter pH-Stat-Bedingungen bei pH 9,0 automatisch mit 0,1 mol/l Natronlauge (D.) mindestens 5 Minuten lang titriert. Es wurde der Laugenverbrauch zwischen der 1. und der 5. Minute ausgewertet. Die Titration wurde je Inhibitorkonzentration zweimal durchgeführt und die Ergebnisse beider Bestimmungen wurden gemittelt. Ebenso wurde die Titration ohne Zugabe von Inhibitorlösung durchgeführt, um die Aktivität der Referenzsuspension zu ermitteln. Um die Lipase-Restaktivität in Bezug auf den Lipase-Referenz-Standard in FIP/Ph.Eur.-Einheiten pro Gramm beim Einsatz von Inhibitoren zu berechnen, wurde folgende Gleichung verwendet:

$$[(n_u \times m_r) / (n_r \times m_u)] \times A = \text{Lipaseeinheiten / g}$$

$n_u$ = ml Verbrauch an 0,1 mol/l Natronlauge / Min. bei der Titration der Lipase-Inhibitor- Testsuspension

$n_r$ = ml Verbrauch an 0,1 mol/l Natronlauge / Min. bei der Titration der Referenzsuspen- sion

$m_u$ = Masse des Lipase-Referenz-Standards in der Testsuspension mit Inhibitor in mg

$m_r$ = Masse des Lipase-Referenz-Standards in der Referenzsuspension in mg

A = deklarierte Aktivität des Referenz-Standards in der Referenzsuspension in FIP/Ph.Eur.-E / g Standard

Das Ergebnis wird in "Prozent Restaktivität" angegeben:

$$\text{Restaktivität [\%]} = \text{Lipase-FIP/Ph.Eur.-E / g} \times 100 / \text{Sollaktivität des Referenz-Standards}$$

Die Sollaktivität des Lipase-Referenz-Standards nach FIP beträgt 36700 Lipase-FIP/Ph.Eur.-E / g.

Als Maß für die inhibitorische Wirksamkeit der Substanzen der Formel I wurde deren $IC_{50}$-Wert entsprechend der vorstehend für den pNpp-Test angegebenen Weise bestimmt. In dem vorstehend angegebenen Pankreaslipase-Aktivitätstest zeigten die in der nachfolgend aufgeführten Tabelle 2 aufgeführten Testsubstanzen der Formel I die nachfolgend angegebenen $IC_{50}$-Werte. Die angegebenen Beispielsnummem beziehen sich auf die nachfolgend angegebenen Herstellungsbeispiele.

Tabelle 2: Schweinepankreaslipase-inhibierende Wirkung der Testsubstanzen in vitro gegenüber Triglyceriden in Olivenöl

| **Beispiel Nr.** | **$IC_{50}$[nM]** |
|---|---|
| 1 | 17 |
| 15 | 75 |

Die Lipase-hemmenden Eigenschaften der Verbindungen der Formel I lassen sich auch durch Fütterungsversuche an der Ratte belegen.

Die Verbindungen der Formel I können in üblichen pharmazeutischen Zubereitungen verabreicht werden. Die zu verwendenden Dosen können individuell verschieden sein und variieren naturgemäß je nach Art des zu behandelnden Zustandes und der verwendeten Substanz. Im allgemeinen eignen sich zur Applikation am Menschen und an größeren Säugetieren jedoch Arzneiformen mit einem Wirkstoffgehalt von 10 bis 500 mg, insbesondere 50 bis 250 mg Wirkstoff pro Einzeldosis.

Die Verbindungen können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt. Diese Präparate können pharmazeutisch übliche anorganische und/oder organische Trägerstoffe, wie z. B. Talkum, Milchzucker oder Stärke, neben pharmazeutisch üblichen Hilfsmitteln, beispielsweise Gleitmitteln oder Tablettensprengmitteln, enthalten. Flüssige Präparate wie Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z. B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkemen verpreßt werden.

Die nachfolgende angegebenen Herstellungsbeispiele zur Herstellung von Verbindungen der allgemeinen Formel I sollen die Erfindung näher beschreiben, ohne sie in ihrem Umfang zu beschränken.

Beispiel 1: trans-(3-Hexyl-4-oxo-oxetan-2-yl)-pentyl-(4-phenoxyphenyl)-rbamat

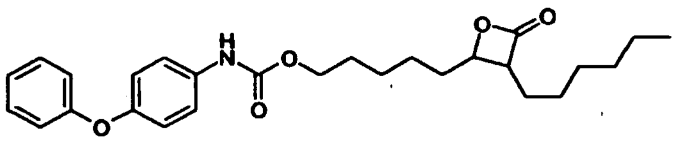

A) Unter einer Stickstoffatmosphäre wurden bei 0° C zu einer Lösung von 41,9 g 1,6-Hexandiol in 400 ml trockenem DMF portionsweise 18,9 g Kalium-tert.-butylat hinzugegeben. Nach 15 Minuten wurde eine Lösung von 14 ml Benzylbromid in 120 ml trockenem DMF bei 0° C hinzugetropft. Das Reaktionsgemisch wurde 5 Minuten lang bei 0° C und anschließend 2 Stunden bei Raumtemperatur gerührt. Der Niederschlag wurde abfiltriert und das Filtrat im Vakuum eingeengt. Der Rückstand wurde in Dichlormethan aufgenommen und nacheinander mit Wasser und gesättigter wäßriger Kochsalzlösung gewaschen. Die organische Phase wurde abgetrennt, über Natriumsulfat getrocknet und im Vakuum eingedampft. Das Rohprodukt wurde mit einem Laufmittelgemisch von n-Hexan/Essigsäureethylester (= EE) (4:1 v/v) an Kieselgel flashchromatographiert, wobei 19 g öliges 6-(Benzyloxy)hexan-1-ol erhalten wurden, $^1$H-NMR (400 MHz, $CDCl_3$): δ = 1,33-1,46 (m, 4H), 1,54-1,68 (m, 4H), 3,47 (t, 2H), 3,63 (t, 2H), 4,50 (s, 2H), 7,24-7,37 (m, 5H).

B) Unter Stickstoffatmosphäre wurde zu einer Lösung von 14,8 ml Oxalylchlorid in 250 ml trockenem Dichlormethan bei -55° C eine Lösung von 25,1 ml DMSO in 50 ml trockenem Dichlormethan hinzugetropft. Nach dreiminütigem

Rühren wurde bei dieser Temperatur langsam eine Lösung von 24 g 6-(Benzyloxy)hexan-1-ol in 100 ml trockenem Dichlormethan hinzugetropft. Nach 15 Minuten wurden 103 ml Triethylamin hinzugetropft. Danach wurde das Reaktionsgemisch 10 Minuten lang bei -55° C gerührt und anschließend auf Raumtemperatur (= RT) erwärmt. Man wusch zuerst mit Wasser und dann mit gesättigter wäßriger Kochsalzlösung. Die organische Phase wurde abgetrennt, über Natriumsulfat getrocknet und im Vakuum eingedampft. Das Rohprodukt wurde zur Reinigung mit einem Laufmittelgemisch von n-Hexan/EE (8:1 v/v) an Kieselgel flashchromatographiert, wobei 15,7 g 6-(Benzyloxy)hexanal erhalten wurden, $^1$H-NMR (400 MHz, $CDCl_3$): δ = 1,38-1,46 (m, 2H), 1,59-1,70 (m, 4H), 2,43 (dt, 1H), 3,47 (t, 2H), 4,49 (s, 2H), 7,24-7,36 (m, 5H), 9,76 (t, 1H).

C) Zu einer Lösung von 23,3 ml Diisopropylamin in 200 ml THF wurden unter Stickstoffatmosphäre bei -70 °C 104,2 ml einer 1,6 molaren Lösung von Butyllithium in n-Hexan hinzugetropft. Nachdem diese Lösung auf -20° C erwärmt wurde und für 10 Minuten bei dieser Temperatur belassen wurde, wurde erneut auf -70 °C abgekühlt und 17,5 ml Essigsäuremethylester langsam hinzugetropft. Nach 15-minütigem Rühren wurde bei dieser Temperatur eine Lösung von 30,16 g 6-(Benzyloxy)hexanal in 250 ml THF hinzugetropft und für 10 Minuten bei -70 °C belassen. Nach langsamer Erwärmung auf -20 °C wurden 150 ml einer gesättigten Ammoniumchloridlösung hinzugegeben und mit Essigsäure-ethylester verdünnt. Nach Abtrennung der organischen Phase wurde die wäßrige Phase zweimal mit EE extrahiert und die vereinigten organischen Auszüge mit Wasser und Kochsalzlösung gewaschen. Nach Trocknung über Natriumsulfat wurde im Vakuum eingeengt und das Rohprodukt mit einem Laufmittelgemisch n-Hexan/EE (9:1 v/v) an Kieselgel flashchromatographiert, wobei nach Eindampfen im Vakuum 25,6 g 8-Benzyloxy-3-hydroxy-octansäure-methylester erhalten wurden, $^1$H-NMR (400 MHz, DMSO-d6): δ = 1,24-1,40 (m, 6H), 1,53 (m, 2H), 2,29 (dd, J=8,2; 14,7 Hz, 1H), 2,39 (dd, J=4,8; 14,7 Hz, 1H), 3,41 (t, 2H), 3,57 (s, 3H), 3,80 (m, 1H), 4,44 (s, 2H), 4,63 (d, J=5,7 Hz, 1H), 7,24-7,37 (m, 5H).

D) Zu einer Lösung von 14,3 ml Diisopropylamin in 100 ml THF wurden unter Stickstoffatmosphäre bei -70 °C 89,4 ml einer 1,6 molaren Lösung von Butyllithium in n-Hexan hinzugetropft. Nachdem diese Lösung auf -20° erwärmt worden und für 10 Minuten bei dieser Temperatur belassen worden war, wurde auf -75°C abgekühlt. Dazu wurde dann eine Lösung von 18,0 g 8-Benzyloxy-3-hydroxy-octansäuremethylester in 15 ml THF langsam hinzugegeben und über 2,5 Stunden auf -45°C erwärmt. Nach erneuter Abkühlung auf -70 °C wurden 13,0 ml HMPT in 10 ml THF hinzugetropft und 30 Minuten gerührt. Dann wurden 30,4 g n-Hexyliodid bei -70 °C über 30 Minuten hinzugetropft. Dieses Reaktionsgemisch ließ man über Nacht auf 10 °C erwärmen. Zur Aufarbeitung wurde der Reihe nach mit verdünnter wäßriger

Kaliumhydrogensulfatlösung und dreimal mit Wasser gewaschen. Anschließend wurde die organische Phase über Natriumsulfat getrocknet und unter vermindertem Druck eingedampft. Dieser Versuch wurde mit 18,0 g 8-Benzyloxy-3-hydroxyoctansäure-methylester wiederholt. Insgesamt wurden 75 g Rohprodukt erhalten, das mit einem Laufmittelgemisch von n-Hexan/EE (9:1 v/v), dessen Zusammensetzung kontinuierlich auf 3:1 verändert wurde, an Kieselgel flashchromatographiert wurde. Es wurden 23,7 g diastereomerenreiner 8-Benzyloxy-3-hydroxy-octansäuremethylester erhalten.

E) 25,33 g 8-Benzyloxy-2-hexyl-3-hydroxy-octansäuremethylester wurden in 180 ml Ethanol gelöst und mit einer Lösung von 5,2 g Lithiumhydroxid-Hydrat in 60 ml Wasser versetzt. Dieses Reaktionsgemisch wurde 48 Stunden lang bei RT gerührt. Dann wurde mit wäßriger Kaliumhydrogensulfatlösung auf pH 6 eingestellt und unter vermindertem Druck eingedampft. Der Rückstand wurde mit EE aufgenommen, und der Reihe nach mit wäßriger Kaliumhydrogensulfatlösung, Wasser und gesättigter wäßriger Kochsalzlösung gewaschen. Die organische Phase wurde abgetrennt, über Natriumsulfat getrocknet, im Vakuum eingedampft und schließlich 4 Stunden lang im Ölpumpenvakuum getrocknet. Man erhielt 23,9 g 8-Benzyloxy-2-hexyl-3-hydroxy-octansäure, IR: 2928, 2856, 1706, 1454, 1100 $cm^{-1}$.

F) Unter Stickstoffatmosphäre wurden 23,9 g 8-Benzyloxy-2-hexyl-3-hydroxy-octansäure in 100 ml Pyridin gelöst und bei -20°C mit 13,3 g Benzolsulfonsäurechlorid versetzt. Dieses Reaktionsgemisch wurde 18 Stunden lang bei 4 °C stehen gelassen. Anschließend nahm man mit EE auf, wusch die organische Phase der Reihe nach dreimal mit Wasser, verdünnter wäßriger Zitronensäurelösung und gesättigter Kochsalzlösung, trocknete über Natriumsulfat und dampfte unter vermindertem Druck ein. Dieser Versuch wurde mit 5,76 g 8-Benzyloxy-2-hexyl-3-hydroxyoctansäure wiederholt. Insgesamt wurden 30 g Rohprodukt erhalten, welches mit einem Laufmittelgemisch aus n-Hexan/EE (3:1 v/v) an Kieselgel flashchromatographiert wurde, wobei insgesamt 21,4 g 4-[5-(Benzyloxy)pentyl]-3-hexyl-oxetan-2-on als reines trans-Produkt erhalten wurden, IR: 2929, 2857, 1818, 1455, 1117 $cm^{-1}$.

G) 10,0 g des vorstehend erhaltenen Produkts wurden in 200 ml EE gelöst und mit 1,0 g eines 10%igen Pd/C-Katalysators versetzt. Anschließend wurde 4 Stunden lang bei 2,5 bar Wasserstoffdruck hydriert. Nach Abfiltrieren vom Katalysator wurde die organische Phase im Vakuum eingedampft und man erhielt 7,1 g 3-Hexyl-4-(5-hydroxypentyl)-oxetan-2-on als reines trans-Produkt, $^1$H-NMR (400 MHz, $CDCl_3$): δ = 0,89 (t, 3H), 1,2-1,9 (m, 18H), 3,17 (m, 1H), 3,66 (t, 2H), 4,22 (m, 1H).

H) Unter Stickstoffatmosphäre wurde zu einer Lösung von 5,8 g 4-Phenoxyphenylisocyanat in 50 ml Dichlormethan bei 0° C eine Lösung von 5,5 g 3-Hexyl-4-(5-hydroxypentyl)-oxetan-2-on in 25 ml Dichlormethan hinzugetropft und 5 Minuten lang bei 0° C belassen. Anschließend wurde 2 Stunden lang bei RT gerührt und zur Vervollständigung der Reaktion wurden noch 0,4 ml Diisopropylethylamin ( = "Hünig-Base") hinzugegeben. Nach Rühren über Nacht wurde das Reaktionsgemisch zunächst mit Wasser, dann mit gesättigter wäßriger Kochsalzlösung gewaschen. Dann wurde die organische Phase über Natriumsulfat getrocknet und im Vakuum eingedampft. Der verbliebene Rückstand wurde an Kieselgel flashchromatographiert, wobei zunächst reines n-Hexan, dem ein stetig steigender Anteil an EE hinzugefügt wurde, als Laufmittel benutzt wurde. Nach Eindampfen der Produktfraktionen im Vakuum erhielt man einen festen Rückstand, der aus n-Hexan/EE umkristallisiert wurde. Es wurden 7,1 g der Titelverbindung als reines trans-Produkt erhalten, Fp. 63,1-66,3°C; $^1$H-NMR (400 MHz, $CDCl_3$): δ = 0,89 (t, 3H), 1,2-1,9 (m, 18H), 3,17 (m, 1H), 4,17 (t, 2H), 4,22 (m, 1H), 6,55 (s, br, 1H), 6,96-7,00 (m, 4H), 7,07 (dt, 1H), 7,28-7,37 (m, 4H).

Beispiel 2: cis/trans-5-(3-Hexyl-4-xo-oxetan-2-yl)pentyl-phenylcarbamat

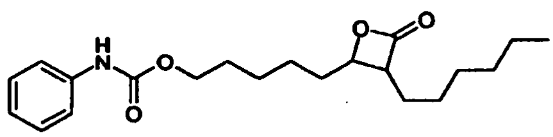

A) Zu einer Lösung von 20,9 g 1,6-Hexandiol in 300 ml DMF wurden 16,3 g Imidazol gegeben. Dazu wurde bei RT eine Lösung von 17,8 g tert.-Butyldimethylsilylchlorid in 120 ml DMF getropft. Anschließend wurde 20 Stunden lang gerührt. Es wurde Wasser hinzugegeben und die wäßrige Phase wurde mehrfach mit EE extrahiert. Die vereinigten organischen Phasen wurden der Reihe nach mit Wasser und mit gesättigter wäßriger Kochsalzlösung gewaschen, über Natriumsulfat getrocknet und im Vakuum eingedampft. Der verbliebene Rückstand wurde mit einem Laufmittelgemisch aus n-Hexan/EE (9:1 v/v) an Kieselgel flashchromtographiert. Man erhielt 17,4 g 6-{[*Tert*-butyl(dimethyl) silyl]oxy}hexan-1-ol, $^1$H-NMR (400 MHz, $CDCl_3$): δ = 0,05 (s, 6H), 0,89 (s, 9H), 1,37 (m, 4H), 1,55 (m, 4H), 3,61 (t, 2H), 3,64 (t, 2H).

B) Unter Stickstoffatmosphäre wurde zu einer Lösung von 9,4 ml Oxalylchlorid in 250 ml trockenem Dichlormethan bei -55 °C eine Lösung von 15,9 ml DMSO in 75 ml trockenem Dichlormethan hinzugetropft. Nach dreiminütigem Rühren wurde bei dieser Temperatur langsam eine Lösung von 17 g 6-{[*Tert*-butyl(dimethyl)Sily]oxy}-hexan-1-ol in 150 ml trockenem Dichlormethan hinzugetropft. Nach 15 Minuten wurden 65,9 ml Triethylamin hinzugetropft. Danach wurde das Reaktionsgemisch 15 Minuten lang bei -55 °C gerührt und anschließend auf RT erwärmt. Die organische Phase wurde der Reihe nach mit Wasser und mit gesättigter wäßriger Kochsalzlösung gewaschen. Die organische Phase wurde abgetrennt, über Natriumsulfat getrocknet, abfiltriert und im Vakuum eingedampft. Der verbliebene Rückstand wurde mit einem Laufmittelgemisch von n-Hexan/EE (9:1 v/v) an Kieselgel flashchromatographiert, wobei 14,9 g 6-{[*Tert*-butyl(dimethyl)silyl]oxy}hexanal erhalten wurden, $^1$H-NMR (400 MHz, $CDCl_3$): δ = 0,04 (s, 6H), 0,89 (s, 9H), 1,38 (m, 2H), 1,54 (m, 2H), 1,65 (m, 2H), 2,43 (dd, J=1,8; 7,3 Hz, 1H), 3,61 (t, 2H), 9,77 (t, J=1,8 Hz, 1H).

C) Zu einer Lösung von 25,5 ml Diisopropylamin in 300 ml THF wurden bei -70 °C 113,4 ml einer 1,6 molaren Lösung von n-Butyllithium in n-Hexan hinzugetropft. Nachdem diese Lösung auf 0 °C erwärmt worden und für 10 Minuten bei dieser Temperatur belassen worden war, wurde auf -50 °C abgekühlt und es wurde bei dieser Temperatur eine Lösung von 14,2 ml Octansäure in 150 ml THF langsam hinzugetropft. Nach 15-minütigem Rühren bei dieser Temperatur wurde für 1 Stunde bei Raumtemperatur gerührt. Dann wurde auf -78 °C abgekühlt und man ließ eine Lösung von 19 g 6-{[*Tert*-butyl(dimethyl)silyl]oxy}hexanal in 150 ml THF so hinzutropfen, daß die Temperatur -60° C nicht überstieg. Nach dreistündigem Rühren bei -78° C wurde auf Raumtemperatur erwärmt. Anschließend wurden 150 ml einer gesättigten wäßrigen Ammoniumchloridlösung hinzugegeben und man extrahierte mehrfach mit EE. Die vereinigten organischen Phasen wurden der Reihe nach mit Wasser und gesättigter wäßriger Kochsalzlösung gewaschen, über Natriumsulfat getrocknet und im Vakuum eingedampft. Der verbliebene Rückstand wurde mit

einem Laufmittelgemisch aus n-Hexan/EE (4:1 v/v) an Kieselgel flashchromatographiert. Eindampfen der Produktfraktionen im Vakuum lieferte 28,5 g 8-{[*Tert*-butyl(dimethyl)silyl]oxy}2-hexyl-3-hydroxy-octansäure als syn/anti-Gemisch, $^1$H-NMR (400 MHz, $CDCl_3$; ausgewählte Signale): δ = 0,05 (s, 6H), 0,88 (t, 3H), 0,89 (s, 9H), 2,47 (m, 1H), 3,61 (t, 2H), 3,72 und 3,85 (m, 1H).

D) Unter Stickstoffatmosphäre wurde zu einer Lösung von 7,6 ml frisch destilliertem Benzolsulfonsäurechlorid in 100 ml Dichlormethan 49 ml Pyridin bei 0 °C hinzugegeben. Anschließend wurde bei dieser Temperatur eine Lösung von 11,1 g 8-{[*Tert*-butyl(dimethyl)silyl]oxy}-2-hexyl-3-hydroxy-octansäure in 100 ml Dichlormethan hinzugetropft. Dieses Reaktionsgemisch wurde für 20 Stunden bei 4 °C belassen. Anschließend wusch man die organische Phase der Reihe nach mit Wasser und gesättigter wäßriger Kochsalzlösung, trocknete über Natriumsulfat und dampfte im Vakuum ein. Der verbliebene Rückstand wurde an Kieselgel flashchromatographiert, wobei reines n-Hexan, dem, ein stetig steigender Anteil an EE hinzugefügt wurde, als Laufmittel benutzt wurde. Man erhielt 4,0 g 4-(5-{[*Tert*-butyl-(dimethyl)silyl]oxy}pentyl)-3-hexyl-oxetan-2-on als trans/cis-Gemisch (7:1), $^1$H-NMR (400 MHz, $CDCl_3$): δ = 0,05 (s, 6H), 0,89 (t, 3H), 0,89 (s, 9H), 3,16 (m, 1H), 3,61 (t, 2H), 4,21 (m, 1H) (ausgewählte Signale für trans-Isomer;); 3,60 (m, 1H), 4,52 (m, 1H) (ausgewählte Signale für cis-Isomer).

E) Unter Stickstoffatmosphäre wurde zu einer Lösung von 3,1 g 4-(5-{[*Tert*-butyl-(dimethyl)-silyl]oxy}pentyl)-3-hexyl-oxetan-2-on in 40 ml THF eine Lösung von 10,97 g Tetrabutylammoniumfluorid-trihydrat in 40 ml THF bei 0 °C hinzugetropft und erst 1 Stunde lang bei dieser Temperatur und anschließend 1 Stunde lang bei RT gerührt. Anschließend wurde das Reaktionsgemisch mit EE verdünnt und die organische Phase der Reihe nach mit Wasser und gesättigter wäßriger Kochsalzlösung gewaschen. Die organische Phase wurde über Natriumsulfat getrocknet und im Vakuum eingedampft. Der verbliebene Rückstand wurde mit einem Laufmittelgemisch aus n-Hexan/EE (9: 1 v/v) an Kieselgel flashchromatographiert. Eindampfen der Produktfraktionen im Vakuum lieferte 1,9 g 3-Hexyl-4-(5-hydroxypentyl)-oxetan-2-on als trans/cis-Gemisch (8:1), $^1$H-NMR (400 MHz, $CDCl_3$): trans: δ = 3,17 (m, 1H), 4,22 (m, 1H); cis: δ = 3,6 (m, 1H), 4,53 (m, 1H) (charakteristische Signale der Protonen des β-Lactons).

F) Unter Stickstoffatmosphäre wurde zu einer Lösung von 0,45 g Phenylisocyanat in 5 ml Dichlormethan bei 0 °C eine Lösung von 0,3 g 3-Hexyl-4-(5-hydroxypentyl)-oxetan-2-on in 5 ml Dichlormethan hinzugetropft. Anschließend wurde 5 Minuten lang bei 0 °C und dann 1 Stunde lang bei RT gerührt. Zur Aufarbeitung wurde die organische Phase der Reihe nach mit Wasser und mit gesättigter wäßriger Kochsalzlösung gewaschen, über Natriumsulfat getrocknet und im Vakuum eingedampft. Der verbliebene Rückstand wurde mit einem Laufmittelgemisch aus n-Hexan/EE (9:1 v/v) an Kieselgel flashchromatographiert. Man erhielt 0,4 g 5-(3-Hexyl-4-oxo-oxetan-2-yl)pentyl-phenylcarbamat als trans/cis-Gemisch, $^1$H-NMR (400 MHz, $CDCl_3$) trans: δ = 3,17 (m, 1H), 4,22 (m, 1H); cis: δ = 3,60 (m, 1H), 4,53 (m, 1H) (charakteristische Signale der Protonen des β-Lactons); IR: 3331, 2931, 2859, 1818, 1732, 1600, 1540 $cm^{-1}$.

Beispiel 3: cis/trans-5-(3-Hexy)-4-oxo-oxetan-2-yl)pentyl-isopropylcarbamat

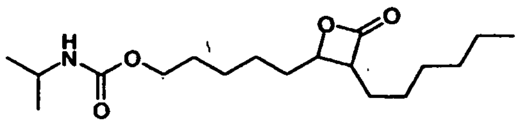

A) Unter Stickstoffatmosphäre wurden zu einer Lösung von 22,8 g 1,6-Hexandiol in 400 ml THF bei 0 °C 12,4 ml 3,4-Dihydro-2*H*-pyran und 0,7 g p-Toluolsulfonsäure hinzugegeben. Nachdem 45 Minuten lang bei 0°C gerührt worden war, ließ man auf RT erwärmen. Anschließend gab man weitere 6,2 ml 3,4-Dihydro-2*H*-pyran hinzu und rührte eine weitere Stunde lang bei RT. Man dampfte im Vakuum ein, nahm den verbliebenen Rückstand mit Dichlormethan auf, wusch die organische Phase der Reihe nach mit gesättigter wäßriger Natriumhydrogencarbonatlösung, Wasser und gesättigter Kochsalzlösung und trocknete über Natriumsulfat. Eindampfen im Vakuum und Flashchromatographie des verbliebenen Rückstandes an Kieselgel mit einem Laufmittelgemisch, das zunächst aus n-Hexan/EE (9:1 v/v) bestand und durch stetig erhöhten Anteil an EE variiert wurde, lieferte 18,3 g 6-[(Tetrahydro-2*H*-pyran-2-yl)oxy]hexan-1-ol, $^1$H-NMR (400 MHz, $CDCl_3$): δ = 1,34-1,47 (m, 4H), 1,48-1,65 (m, 8H), 1,71 (m, 1H), 1,83 (m, 1H), 3,40 (dt, J=6,6; 9,6 Hz, 1H), 3,50 (m, 1H), 3,65 (t, J=6,5 Hz, 2H), 3,74 (dt, J=6,8; 9,6 Hz, 1H), 3,87 (m, 1H), 4,57 (dd, J=2,7; 4,2 Hz, 1H).

B) Unter Stickstoffatmosphäre wurde zu einer Lösung von 11,6 ml Oxalylchlorid in 250 ml trockenem Dichlormethan bei -55 °C eine Lösung von 19,7 ml DMSO in 75 ml trockenem Dichlormethan hinzugetropft. Nach fünfminütigem Rühren wurde bei dieser Temperatur langsam eine Lösung von 18,3 g 6-[(Tetrahydro-2*H*-pyran-2-yl)oxy]hexan-1-ol in 150 ml trockenem Dichlormethan hinzugetropft. Nach 15 Minuten wurden 81,5 ml Triethylamin hinzugetropft. Danach wurde das Reaktionsgemisch 15 Minuten lang bei -55 °C gerührt und anschließend auf RT aufgetaut. Man wusch die organische Phase zuerst mit Wasser und dann mit gesättigter wäßriger Kochsalzlösung, trennte von der wäßrigen Phase und trocknete die organische Phase über Natriumsulfat. Man filtrierte vom Trockenmittel ab, dampfte im Vakuum ein und flashchromatographierte den verbliebenen Rückstand an Kieselgel mit einem Laufmittelgemisch von n-Hexan/EE (9:1 v/v), dessen Gehalt an EE stetig erhöht wurde. Man erhielt 14,1 g 6-[(Tetrahydro-2*H*-pyran-2-yl)oxy]hexanal, $^1$H-NMR (400 MHz, $CDCl_3$): $\delta$ = 1,37-1,47 (m, 2H), 1,48-1,75 (m, 9H), 1,77-1,89 (m, 1H), 2,44 (dt, J=1,8; 7,3 Hz, 2H), 3,39 (dt, J=6,4; 9,7 Hz, 1H), 3,50 (m, 1H), 3,74 (dt, J=6,7; 9,7 Hz, 1H), 3,86 (m, 1H), 4,56 (dd, J=2,8; 4,4 Hz, 1H), 9,77 (t, 1,8 Hz, 1H).

C) Zu einer Lösung von 21,6 ml Diisopropylamin in 250 ml THF wurden unter Stickstoffatmosphäre bei -70 °C 96,1 ml einer 1,6 molaren Lösung von n-Butyllithium in n-Hexan hinzugetropft. Nachdem diese Lösung auf 0 °C erwärmt worden und für 10 Minuten bei dieser Temperatur belassen worden war, wurde auf -50° C abgekühlt und es wurde bei dieser Temperatur eine Lösung von 12,1 ml Octansäure in 125 ml THF langsam hinzugetropft. Nach 15-minütigem Rühren bei dieser Temperatur wurde für 1 Stunde bei RT gerührt. Dann wurde auf -78° C abgekühlt und man ließ eine Lösung von 14 g 6-[(Tetrahydro-2*H*-pyran-2-yl)oxy]hexanal in 125 ml THF hinzutropfen. Nach dreistündigem Rühren bei -78° C wurde auf RT erwärmt. Anschließend wurden 150 ml einer gesättigten wäßrigen Ammoniumchloridlösung hinzugegeben und es wurde mehrfach mit EE extrahiert. Die vereinigten organischen Phasen wurden der Reihe nach mit Wasser und mit gesättigter wäßriger Kochsalzlösung gewaschen, über Natriumsulfat getrocknet und im Vakuum eingedampft. Der verbliebene Rückstand wurde mit einem Laufmittelgemisch aus n-Hexan/EE (4: 1 v/v) an Kieselgel flashchromatographiert, wobei der Anteil an EE stetig erhöht wurde. Eindampfen der Produktfraktionen im Vakuum lieferte 16,2 g 2-Hexyl-3-hydroxy-8-[(tetrahydro-2*H*-pyran-2-yl)oxy]octansäure als syn/anti-Gemisch, $^1$H-NMR (400 MHz, $CDCl_3$): $\delta$ = 0,88 (m, 3H), 1,25-1,89 (m, 25H), 2,47 (m, 1H), 3,40 (dt, 1H), 3,51 (m, 1H), 3,73 (m,1H), 3,86 (m, 1H), 4,57 (m, 1H).

D) Unter Stickstoffatmosphäre wurde zu einer Lösung von 12 ml frisch destilliertem Benzolsulfonsäurechlorid in 150 ml Dichlormethan 50 ml Pyridin bei 0 °C hinzugegeben. Anschließend wurde bei dieser Temperatur eine Lösung von 16,2 g 2-Hexyl-3-hydroxy-8-[(Tetrahydro-2*H*-pyran-2-yl)oxy]octansäure in 100 ml Dichlormethan hinzugetropft. Dieses Reaktionsgemisch wurde für 20 Stunden bei 4 °C belassen. Anschließend wusch man die organische Phase der Reihe nach mit Wasser und gesättigter wäßriger Kochsalzlösung, trocknete über Natriumsulfat und dampfte im Vakuum ein. Der verbliebene Rückstand wurde an Kieselgel flashchromatographiert, wobei reines n-Hexan, dem ein stetig steigender Anteil an EE (bis 8:2 v/v) hinzugefügt wurde, als Laufmittel benutzt wurde. Man erhielt 10,0 g 3-Hexyl-4-{5[(Tetrahydro-2*H*-pyran-2-yl)oxy]pentyl}oxetan-2-on als Diastereomerengemisch (2:1), $^1$H-NMR (400 MHz, $CDCl_3$) trans: $\delta$ = 3,16 (m, 1H), 4,21 (m, 1H); cis: $\delta$ = 3,59 (m, 1H), 4,53 (m, 1H) (charakteristische Signale der Protonen des β-Lactons), IR: 1822, 1465, 1077 $cm^{-1}$.

E) 1,89 g 3-Hexyl-4-{5[(Tetrahydrd-2*H*-pyran-2-yl)oxy]pentyl}oxetan-2-on wurden in 58 ml Ethanol gelöst und mit 1,46 g Pyridinium-p-toluolsulfonat versetzt. Man rührte 4 Stunden lang bei 50 °C und gab dann weitere 1,46 g Pyridinium-p-toluolsulfonat hinzu. Dieses Reaktionsgemisch wurde 1 Stunde lang bei 50 °C und dann 20 Stunden lang bei RT gerührt. Man filtrierte vom Niederschlag ab, dampfte das Filtrat im Vakuum ein und flashchromatographierte den verbliebenen Rückstand an Kieselgel mit einem Laufmittelgemisch aus n-Hexan/EE (9:1 v/v), dessen Anteil an EE kontinuierlich erhöht wurde. Eindampfen der Produktfraktionen lieferte 1,0 g cis/trans-3-Hexyl-4-(5-hydroxypentyl)-oxetan-2-on, welches ohne weitere Reinigung direkt für die nächste Umsetzung eingesetzt wurde.

F) Unter Stickstoffatmosphäre wurde zu einer Lösung von 0,4 g Isopropylisocyanat in 5 ml Dichlormethan bei 0 °C eine Lösung von 0,3 g 3-Hexyl-4-(5-hydroxypentyl)-oxetan-2-on in 5 ml Dichlormethan hinzugetropft. Anschließend wurde 5 Minuten lang bei 0 °C und dann 1 Stunde lang bei RT gerührt. Man fügte 0,2 ml Düsopropylethylamin hinzu und ließ weitere 2 Stunden lang bei 50°C rühren. Man wusch mit. Wasser und gesättigter wäßriger Kochsalzlösung, trocknete über Natriumsulfat und dampfte im Vakuum ein. Der verbliebene Rückstand wurde mit einem Laufmittelgemisch aus n-Hexan/EE (9:1 v/v), dessen Anteil an EE kontinuierlich bis auf 100 % erhöht wurde, an Kieselgel flashchromatographiert. Man erhielt 0,1 g 5-(3-Hexyl-4-oxo-oxetan-2-yl)pentyl-isopropylcarbamat als trans/cis-Gemisch (2:1), $^1$H-NMR (400 MHz, $CDCl_3$): trans: $\delta$ = 3,16 (m, 1H), 4,21 (m, 1H); cis: $\delta$ = 3,60 (m, 1H), 4,52 (m, 1H) (charakteristische Signale der Protonen des β-Lactons); IR: 3321, 2930, 1813, 1687, 1538, 1468 $cm^{-1}$.

Beispiel 4: trans-5- (3-Hexyl-4-oxo-oxetan-2-yl)butyl-benzylcarbamat

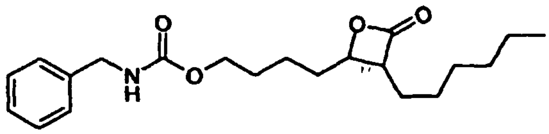

A) Unter Stickstoffatmosphäre wurden 50,0 g 2-Mercaptopyridin, gelöst in 1 l Dichlormethan, bei 0 °C mit 94,0 ml Triethylamin versetzt. Dazu wurden 73,2 g Octanoylchlorid in 200 ml Dichlormethan innerhalb von 10 Minuten hinzugegeben. Nach Entfernung der Kühlung wurde für 90 Minuten bei RT gerührt und anschließend mit 600 ml Wasser versetzt. Nach Abtrennung der organischen Phase wurde die wäßrige Phase dreimal mit je 200 ml Dichlormethan extrahiert. Die vereinigten organischen Phasen wurden über Magnesiumsulfat getrocknet und anschließend im Vakuum eingedampft. Der verbliebene Rückstand wurde mit n-Pentan/Diethylether (1:1 v/v) an Kieselgel flashchromatographiert. Man erhielt 101,6 g Thiooctansäure-S-pyridin-2-yl-ester, $^1$H-NMR (400 MHz, $CDCl_3$): $\delta$ = 0,88 (t, J=6,9 Hz, 3H), 1,23-1,40 (m, 8H), 1,69-1,76 (m, 2H), 2,70 (t, J=7,5 Hz, 2H), 7,28 (ddd, J=7,5; 4,8; 1,1 Hz, 1H), 7,61 (dt, J=0,8; 7,8 Hz, 1H), 7,73 (dt, J=1,9; 7,7 Hz, 1H), 8,62 (ddd, J= 4,8; 1,8; 0,7 Hz, 1H).

B) 97,6 ml einer Lithiumhexamethyldisilazid-Lösung (1M in THF) in 420 ml THF wurden unter Stickstoffatmosphäre auf -10°C abgekühlt, nacheinander mit 12,6 ml DMF und 22,7 ml Triethylamin versetzt und 10 Minuten lang gerührt. Dann wurden 24,5 g Triethylsilylchlorid in 50 ml THF hinzugegeben und die Reaktionsmischung auf -78°C abgekühlt. Anschließend wurden 19,3 g Thiooctansäure-S-pyridin-2-yl-ester in 50 ml THF langsam hinzugetropft und das Gemisch für 1 Stunde bei -78°C gerührt. Nach Entfernung der Kühlung ließ man das Reaktionsgemisch innerhalb einer Stunde auf 10°C erwärmen und versetzte mit 800 ml Wasser. Nach mehrfacher Extraktion mit Diethylether wurde die organische Phase über Magnesiumsulfat getrocknet und im Vakuum eingedampft. Der verbliebene Rückstand wurde mit einem Laufmittelgemisch aus n-Pentan/Diethylether (9:1 v/v) schnell an Kieselgel flashchromatographiert. Eindampfen der Produktfraktionen lieferte 25,6 g 2-({(1E)-1-[(Triethylsilyl)oxy]oct-1-en-1-yl}thio) pyridin, $^1$H-NMR (400 MHz, $CDCl_3$): $\delta$ = 0,60-0,69 (m, 6H), 0.84-0,91 (m, 12H), 1.24-1,43 (m, 8H), 2,18 (q, J=7,2 Hz, 2H), 5,38 (t, J=7,3 Hz, 1H), 6,99 (ddd, J=7,4; 4,9; 1,0 Hz, 1H), 7,34 (dt, J=1,0; 8,1 Hz, 1H), 7,54 (ddd, J=8,1; 7,4; 1,9; Hz, 1H), 8,42 (ddd, J= 4,9; 1,9; 0,9 Hz, 1H).

C) Zu einer Suspension von 34,0 g wasserfreiem Zinkchlorid in 800 ml Dichlormethan wurden bei RT und unter Stickstoffatmosphäre 37,9 g 5-{[*Tert*-butyl(dimethyl)silyl]-oxy}pentanal (Herstellung siehe Beispiel 2B)) hinzugegeben und 15 Minuten lang gerührt. Anschließend wurden 55,9 g 2-({(1E)-1-[(Triethylsilyl)oxy]oct-1-en-1-yl}thio)pyridin hinzugefügt und das Reaktionsgemisch wurde 40 Stunden lang bei RT gerührt. Anschließend gab man gesättigte wäßrige Natriumhydrogencarbonatlösung hinzu, trennte die Phasen und extrahierte die wäßrige Phase dreimal mit Dichlormethan. Die vereinigten organischen Phasen wurden über Magnesiumsulfat getrocknet und im Vakuum auf ein Volumen von ca 500 ml eingedampft. Diese Lösung wurde mit 47,7 g Kupferdibromid versetzt und 90 Minuten lang bei RT gerührt. Anschließend wurde die entstandene Suspension über Kieselgel filtriert, wobei mit Dichlormethan gespült wurde. Eindampfen im Vakuum und Flashchromatographie des verbliebene Rückstandes an Kieselgel mit einem Laufmittelgemisch aus n-Pentan/Diethylether (33:1 v/v) lieferte 17,8 g 4-(5-{[*Tert*-butyl(dimethyl)silyl] oxy}-butyl)-3-hexyl-oxetan-2-on als reines trans-Produkt, $^1$H-NMR (400 MHz, $CDCl_3$): $\delta$ = 0,04 (s, 6H), 0,85-0,90 (m, 12H), 1,22-1,92 (m, 16H), 3,16 (ddd, J=8,8; 6,5; 3,9 Hz, 1H), 4,21 (ddd, J=7,2; 6,1; 4,0 Hz, 1H).

D) In einem PET-Gefäß wurden 17,8 g der vorstehend erhaltenen Verbindung in 200 ml THF gelöst, mit 10 ml HF-Pyridin-Komplex versetzt und anschließend über Nacht bei RT gerührt. Dann wurde die Reaktionsmischung über Kieselgel filtriert und mit Diethylether gespült. Das Lösungsmittel wurde im Vakuum eingedampft und der verbliebene Rückstand wurde mit einem Laufmittelgemisch aus n-Pentan/Diethylether (1:1 v/v) an Kieselgel flashchromatographiert. Trocknung der Produktfraktionen lieferte 10,6 g 3-Hexyl-4-(5-hydroxybutyl)-oxetan-2-on als reines trans-Produkt, $^1$H-NMR (400 MHz, $CDCl_3$): $\delta$ = 0,89 (t, J=6,3 Hz, 3H), 1,29-1,93 (m, 16H), 2,14 (s br, 1H), 3,19 (ddd, J=8,6; 6,6; 3,9 Hz, 1H), 3,65 (t, J=6,3 Hz, 2H), 4,24 (ddd, J=7,4; 5,6; 4,0 Hz, 1H).

E) Unter Stickstoffatmosphäre wurde zu einer Lösung von 0,26 g Benzylisocyanat in 5 ml Dichlormethan bei 0°C eine Lösung von 0,3 g 3-Hexyl-4-(5-hydroxybutyl)-oxetan-2-on in 5 ml Dichlormethan hinzugetropft. Anschließend wurde 10 Minuten lang bei 0°C und dann 20 Stunden lang bei RT gerührt. Man wusch die organische Phase der

Reihe nach mit Wasser und gesättigter wäßriger Kochsalzlösung, trocknete über Natriumsulfat und dampfte im Vakuum ein. Der verbliebene Rückstand wurde mit einem Laufmittelgemisch aus n-Hexan/EE (3:2 v/v) an Kieselgel flashchromatographiert und die vereinigten Produktfraktionen wurden anschließend aus einem Gemisch von n-Hexan/EE umkristallisiert. Man erhielt 0,4 g der Titelverbindung als reines trans-Produkt, Fp. 50,9 - 52,5°C; $^1$H-NMR (400 MHz, $CDCl_3$): δ = 0,89 (t, 3H), 3,17 (m, 1H), 4,12 (t, 2H), 4,20 (m, 1H), 4,36 (d, 2H), 4,94 (s br, 1H), 7,23-7,37 (m, 5).

Beispiel 5 trans-5-(3-Benzyl-4-xo-oxetan-2-yl)pentyl-(4-acetylphenyl)carbamat

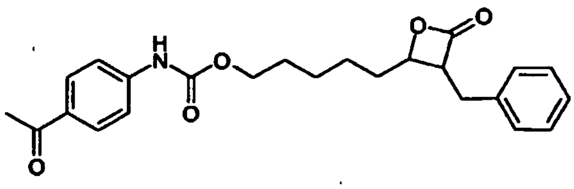

A) Zu einer Lösung von 2,2 g Diisopropylamin in 15 ml THF wurden bei -60 °C 13,7 ml einer 1,6 M n-Butyllithiumlösung in n-Hexan hinzugetropft. Man ließ für 30 Minuten auf -40°C auftauen und kühlte dann auf -75 °C ab. Zu diesem Reaktionsgemisch wurde langsam eine Lösung von 2,8 g 8-Benzyloxy-3-hydroxy-octansäure-methylester (Herstellung siehe Beispiel 1 C)) hinzugetropft und dann bei -55 °C 3 Stunden lang gerührt. Man kühlte auf -75°C, gab 2 ml HMPT hinzu und rührte erneut 30 Minuten lang. Anschließend wurden 3,42 g Benzylbromid, gelöst in 3 ml THF, langsam hinzugetropft und es wurde über einen Zeitraum von 20 Stunden auf RT erwärmt. Man nahm mit EE auf und wusch die organische Phase nacheinander mit Wasser, wäßriger Kaliumhydrogensulfatlösung und erneut Wasser und trocknete anschließend über Natriumsulfat. Der verbliebene Rückstand wurde mit einem Gemisch aus n-Hexan/EE (9:1 v/v, kontinuierlich bis auf 3:1 v/v verändert) an Kieselgel flashchromatographiert. Trocknen der Produktfraktionen lieferte 2,7 g diastereomerenreinen 2-Benzyl-8-(benzyloxy)-3-hydroxy-octansäuremethylester, $^1$H-NMR (200 MHz, $CDCl_3$): δ = 1,2-1,7 (m, 8H), 2,57 (d, 1H), 2,72 (ddd, 1H), 2,90-3,10 (m, 2H), 3,45 (t, 2H), 3,61 (s, 3H), 4,49 (s, 2H).

B) Eine Lösung von 10,8 g 2-Benzyl-8-(benzyloxy)-3-hydroxy-octansäuremethylester in 90 ml Ethanol wurde mit einer Lösung von 1,83 g Lithiumhydroxid in 30 ml Wasser versetzt. Nachdem dieses Reaktionsgemisch 18 Stunden bei RT gerührt worden war, wurde mit 1 M Zitronensäure versetzt und überschüssiges Lösungsmittel im Vakuum eingedampft. Der verbliebene Rückstand wurde in EE aufgenommen, die organische Phase der Reihe nach mit wäßriger Kaliumhydrogensulfatlösung und Wasser gewaschen und schließlich über Natriumsulfat getrocknet. Eindampfen im Vakuum lieferte einen festen Rückstand, welcher aus n-Hexan/EE (1:1 v/v) umkristallisiert wurde. Man erhielt 9,31 g diastereomerenreine 2-Benzyl-8-(benzyloxy)-3-hydroxy-octansäure, Fp. 66-67°C; IR: 3233, 2932, 2849, 1706, 1454, 1125 $cm^{-1}$.

C) Unter Stickstoffatmosphäre wurden 9,31 g 2-Benzyl-8-(benzyloxy)-3-hydroxyoctansäure in 52 ml Pyridin gelöst und bei -20 °C mit 5,1 g Benzolsulfonsäurechlorid versetzt. Danach wurde 2 Stunden lang bei 0 °C und anschließend 48 Stunden lang bei 4°C gerührt. Man verdünnte das Reaktionsgemisch mit Diethylether, wusch der Reihe nach dreimal mit Wasser und je einmal mit 1M Zitronensäure und Wasser und trocknete die organische Phase über Natriumsulfat. Man dampfte das Lösungsmittel im Vakuum ein und flashchromatographierte den verbliebenen Rückstand anschließend an Kieselgel mit einem Laufmittelgemisch aus n-Hexan/EE (zunächst 9:1 v/v, dann stetig auf 3:1, v/v verändert). Trocknen der Produktfraktionen lieferte 7,2 g trans-3-Benzyl-4-[5-(benzyloxy)pentyl]oxetan-2-on, IR: 2933, 2858, 1816, 1454, 1114 $cm^{-1}$.

D) 7,1 g des vorstehend erhaltenen Produkts wurden in 200 ml EE gelöst und mit 0,7 g eines 10%-igen Pd/C-Katalysators versetzt. Anschließend wurde 5 Stunden bei 2,5 bar Wasserstoffdruck hydriert. Nach Abfiltrieren vom Katalysator wurde im Vakuum eingedampft. Man erhielt 4,9 g 3-Benzyl-4-(5-hydroxypentyl)oxetan-2-on, $^1$H-NMR (400 MHz, $CDCl_3$): δ = 1,08-1,34 (m, 5H), 1,47 (m, 2H), 1,62 (m, 1H), 1,80 (m, 1H), 3,00 (dd, J=9,4; 14,3 Hz, 1H), 3,18 (dd, J=5,7; 14,3 Hz, 1H), 3,46 (m, 1H), 3,58 (t, 2H), 4,28 (m, 1H), 7,19 (m, 2H), 7,26 (m, 1H), 7,32 (m, 2H).

E) Unter Stickstoffatmosphäre wurde zu einer Lösung von 0,29 g 4-Acetylphenylisocyanat in 10 ml Dichlormethan bei 0 °C eine Lösung von 0,3 g 3-Benzyl-4-(5-hydroxypentyl]oxetan-2-on in 5 ml Dichlormethan hinzugetropft.

Anschließend wurde 30 Minuten lang bei 0 °C und anschließend 2 Stunden lang bei RT gerührt. Man gab 0,21 ml Diisopropylethylamin hinzu und ließ 2 Tage lang bei RT rühren. Dann wurde mit Dichlormethan aufgenommen, die organische Phase der Reihe nach mit Wasser und gesättigter wäßriger Kochsalzlösung gewaschen, über Natriumsulfat getrocknet und im Vakuum eingedampft. Der verbliebene Rückstand wurde mit einem Laufmittelgemisch aus n-Hexan/EE (3:2 v/v) an Kieselgel flashchromatographiert. Trocknen der Produktfraktionen lieferte 0,4 g der Titelverbindung, Fp. 104-106°C; $^1$H-NMR (400 MHz, $CDCl_3$): $\delta$ = 1,1-1, 4 (m, 4H), 1.54-1,68 (m, 3H), 1,80 (m, 1H), 2,56 (s, 3H), 3,00 (dd, J=9,5; 14,3 Hz, 1H), 3,18 (dd, J=5,7; 14,3 Hz, 1H), 3,47 (m, 1H), 4,12 (t, 2H), 4,28 (m, 1H), 6,84 (s br, 1H), 7,19 ("d", 2H), 7,25 (m, 1H), 7,32 ("t", 2H), 7,48 (d, 2H), 7,93 ("d", 2H).

Beispiel 6: (2S,3S)-5-(3-Hexyl-4-oxo-oxetan-2-yl)-pentyl-(4-phenoxyphenyl)-carbamat

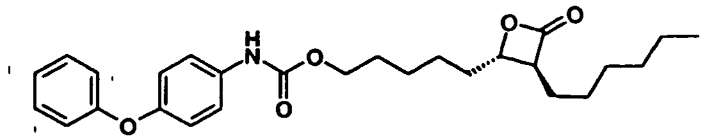

A) Zu einer Lösung von 17,7 g 8-Benzyloxy-3-hydroxy-octansäuremethylester (Herstellung siehe Beispiel 1C)) in 350 ml Dichlormethan wurden erst 14,9 g Pyridiniumchlorochromat und dann etwas Celite® hinzugegeben. Man rührte über Nacht bei RT, gab weitere 15,0 g Pyridiniumchlorochromat hinzu und erhitzte 10 Minuten lang unter Rückflußkühlung zum Sieden. Anschließend wurde wiederum über Nacht bei RT gerührt, dann wurden 400 ml Diethylether zugegeben und es wurde über Celite® filtriert. Das Filtrat wurde im Vakuum eingedampft und der verbliebene Rückstand mit einem Laufmittelgemisch aus n-Heptan/EE (2:1 v/v) an Kieselgel flashchromatographiert. Trocknen der Produktfraktionen lieferte 13,0 g 8-Benzyloxy-3-oxo-octansäuremethylester.

B) Unter Argonatmosphäre wurden zu 0,26 g (S)-(-)2,2'-Bis-(diphenylphosphino)-1,1'-binaphthyl 0,106 g Benzolruthenium(II)-chlorid-Dimer hinzugegeben. Dazu wurden 8 ml DMF gegeben, welches zuvor durch dreimaliges Einfrieren und Auftauen gereinigt worden war. Das entstandene Reaktionsgemisdiwurde 15 Minuten lang auf 115 °C erhitzt und nach Abkühlen auf RT bei 65 °C im Vakuum eingedampft. Zu dem verbliebenen braunen Feststoff gab man eine Lösung von 13 g 8-Benzyloxy-3-oxo-octansäuremethylester in 35 ml Methanol hinzu und überführte die Reaktionslösung dann mit einer Spritze in einen Autoklav. Man hydrierte bei 70 °C 6 Stunden lang bei einem Wasserstoffdruck von 4,2 bar, dampfte anschließend im Vakuum ein und flashchromatographierte den verbliebenen Rückstand mit einem Laufmittelgemisch aus n-Heptan/EE (2:1 v/v) an Kieselgel. Trocknen der Produktfraktionen lieferte 10,7 g (S)-8-Benzyloxy-3-hydroxy-octansäuremethylester als schwach gelbliches Öl. Die Enantiomerenreinheit wurde mit einer Chiralpak AD-H-Säule von Daicel bestimmt. Als mobile Phase wurde ein Laufmittelgemisch aus n-Heptan/tsopropanol (9:1 v/v) verwendet; die Flussrate betrug 1ml/min. Unter diesen Bedingungen wurde eine Retentionszeit von 32,297 Minuten gemessen.

C) Zu einer Lösung von 7,7 g Diisopropylamin in 60 ml THF wurden bei -60 °C 48 ml einer 1,6 M Lösung von n-Butyllithium in n-Hexan hinzugetropft. Man ließ für 30 Minuten auf -40 °C auftauen und kühlte dann auf -78°C ab. Zu dem entstandenen Reaktionsgemisch wurde langsam eine Lösung von 9,8 g (S)-8-Benzyloxy-3-hydroxy-octansäuremethylester in 10 ml THF hinzugetropft und es wurde während 3 Stunden auf -40 °C erwärmt. Dann wurde erneut auf -75°C abgekühlt und es wurden zunächst 8 ml DMPH, gelöst in 8 ml THF, und dann 14,8 g n-Hexyliodid hinzugetropft. Das entstandene Reaktionsgemisch ließ man über Nacht auf RT auftauen, verdünnte es mit EE und wusch die organische Phase der Reihe nach mit Wasser, verdünnter wäßriger Kaliumhydrogensulfatlösung und gesättigter wäßriger Kochsalzlösung. Die organische Phase wurde über Natriumsulfat getrocknet, im Vakuum eingedampft und der verbliebene Rückstand wurde an Kieselgel mit einem Laufmittelgemisch aus n-Hexan/EE (9:1 v/v), welchem allmählich bis zu einer Zusammensetzung von 3:1 (v/v) EE hinzugefügt wurde, flashchromatographiert. Trocknung der Produktfraktionen lieferte 4,0 g (S,S)-8-Benzyloxy-2-hexyl-3-hydroxy-octansäure-methylester, $[\alpha]_D$ = -12,60° (c = 25,7 mg in 2 ml Methanol); IR: 2928, 2856, 1733, 1454, 1164, 1100 $cm^{-1}$.

D) 5,5 g (S,S)-8-Benzyloxy-2-hexyl-3-hydroxy-octansäuremethylester wurden in 40 ml Ethanol gelöst, mit 0,97 g Lithiumhydroxid-Hydrat in 13 ml Wasser versetzt und 24 Stunden lang, bei RT gerührt. Anschließend wurde mit verdünnter wäßriger Kaliumhydrogensulfatlösung versetzt und überschüssiges Lösungsmittel im Vakuum eingedampft. Man nahm mit EE auf, wusch die organische Phase der Reihe nach mit verdünnter wäßriger Kaliumhydrogensulfatlösung und Wasser, trennte von der wäßrigen Phase ab undtrocknete über Natriumsulfat. Nach Eindampfen im Vakuum und Trocknen im Ölpumpenvakuum erhielt man 5,4 g (S,S)-8-Benzyloxy-2-hexyl-3-hydroxyoctansäure als schwach gelbliches Öl, welches ohne weitere Reinigung für die nächste Umsetzung eingesetzt wurde, $[\alpha]_D$ = -14,23° (c = 21,5 mg in 2 ml Methanol); IR: 2928, 2856, 1706, 1454, 1100 $cm^{-1}$.

E) 5,4 g (S,S)-8-Benzyloxy-2-hexyl-3-hydroxy-octansäure wurden in 30 ml Pyridin gelöst und bei -20 °C mit 2,99 g Benzolsulfonsäurechlorid versetzt. Innerhalb von 2 Stunden wurde auf 0 °C erwärmt und anschließend 48 Stunden lang bei 4 °C belassen. Man verdünnte mit Diethylether und wusch die organische Phase der Reihe nach dreimal mit eiskaltem Wasser, 0,5 M Zitronensäure und gesättigter wäßriger Kochsalzlösung. Die organische Phase wurde über Natriumsulfat getrocknet und im Vakuum eingedampft. Der verbliebene Rückstand wurde mit einem Laufmittelgemisch aus n-Hexan/EE (zunächst 9:1 v/v, dann stetig auf 3:1 v/v verändert) an Kieselgel flashchromatographiert. Trocknen der Produktfraktionen lieferte 2,6 g (S,S)-4-[5-(Benzyloxy)pentyl]-3-hexyl-oxetan-2-on, $[\alpha]_D$ = -23,21° (c = 16,6 mg in 2 ml Chloroform), IR: 2929, 2857, 1818, 1455, 1117 $cm^{-1}$.

F) 2,5 g (S,S)-4-[5-(Benzyloxy)pentyl]-3-hexyl-oxetan-2-on wurden in 100 ml EE gelöst und mit 0,25 g eines 10%-igen Pd/C-Katalysator versetzt. Anschließend wurde bei einem Wasserstoffdruck von 2,5 bar 5 Stunden lang hydriert. Man filtrierte vom Katalysator ab, dampfte überschüssiges Lösungsmittel im Vakuum ein und trocknete anschließend im Ölpumpenvakuum. Man erhielt 1,86 g (S,S)-4-[5-Hydroxypentyl]-3-hexyl-oxetan-2-on als Öl, $[\alpha]_D$ = -37,5° (c = 1 in EE).

G) Unter Stickstoffatmosphäre wurde zu einer Lösung von 0,78 g 4-Phenoxyphenylisocyanat in 10 ml Dichlormethan bei 0 °C eine Lösung von 0,6 g (S,S)-4-[5-Hydroxypentyl]-3-hexyl-oxetan-2-on in 10 ml Dichlormethan hinzugetropft. Anschließend wurde 15 Minuten lang bei 0 °C und anschließend 2 Stunden lang bei RT gerührt. Man gab 0,43 ml Diisopropylethylamin hinzu und ließ 2 Tage lang bei RT rühren. Dann wurde mit Dichlormethan aufgenommen, die organische Phase der Reihe nach mit Wasser und gesättigter wäßriger Kochsalzlösung gewaschen, über Natriumsulfat getrocknet und im Vakuum eingedampft. Der verbliebene Rückstand wurde mit einem Laufmittelgemisch aus n-Hexan/EE (4:1 v/v) an Kieselgel flashchromatographiert. Trocknen der Produktfraktionen lieferte 0,74 g der Titelverbindung, Fp. 73-74 °C; $[\alpha]_D$ = -21,5° (c = 1 in EE); $^1$H-NMR (400 MHz, $CDCl_3$): δ 0,89 (t, 3H), 1,2-1,9 (m, 18H), 3,18 (m, 1H), 4,17 (t, 2H), 4,22 (m, 1H), 6,55 (s, br, 1H), 6,96-7,00 (m, 4H), 7,07 ("t", 1H), 7,28-7,37 (m, 4H).

Nach den in den vorstehend angegebenen Beispielen beschriebenen oder nach hierzu analogen Verfahren können auch die in der nachfolgenden Tabelle 3 angegebenen Verbindungen der Formel I hergestellt werden:

Tabelle 3: Weitere Verbindungen der Formel I

| **Bsp.- Nr.** | **$R^1$** | **$R^2$** | **n** | **trans : cis** |
|---|---|---|---|---|
| **7** | Phenyl | n-Hexyl | 3 | cis |
| **8** | Benzyl | n-Hexyl | 5 | 3:1 |
| **9** | 4-Ethoxyphenyl | n-Hexyl | 5 | 3:1 |
| **10** | Benzoyl | n-Hexyt | 5 | 3 : 1 |
| **11** | Phenylethyl | n-Hexyl | 5 | 6:1 |
| **12** | n-Butyl | n-Hexyl | 5 | 2:1 |
| **13** | 3,4-Dichlorphenyl | n-Hexyl | 5 | 12:1 |
| **14** | 4-Fluorphenyl | n-Hexyl | 5 | 3,5:1 |
| **15** | 4-Phenoxyphenyl | n-Hexyl | 4 | trans |
| **16** | 2-Isopropylphenyl | n-Hexyl | 4 | trans |
| **17** | 2-Nitrophenyl | n-Hexyl | 4 | trans |
| **18** | 4-Benzytphenyl | n-Hexyl | 4 | trans |
| **19** | 2-Ethyloxycarbonylphenyl | Benzyl | 5 | trans |
| **20** | Octadecanyl | n-Hexyl | 4 | trans |
| **21** | 2-Trifluormethylphenyl | n-Hexyl | 4 | trans |

(fortgesetzt)

| Bsp.- Nr. | $R^1$ | $R^2$ | n | trans : cis |
|---|---|---|---|---|
| **22** | 4-Heptyloxyphenyl | n-Hexyl | 4 | trans |
| **23** | 2,5-Dimethoxyphenyl | Benzyl | 5 | trans |

alle in Tabelle 3 aufgeführten Verbindungen sind racemisch.

**Beispiel I:** trans-(3-Hexyl-4-oxo-oxetan-2-yl)-pentyl-(4-phenoxyphenyl)-carbamat enthaltende Kapseln:

Es werden Kapseln in folgender Zusammensetzung pro Kapsel hergestellt:

| | |
|---|---|
| trans-(3-Hexyl-4-oxo-oxetan-2-yl)-pentyl-(4-phenoxyphenyl)-carbamat | 20 mg |
| Maisstärke | 60 mg |
| Milchzucker | 300 mg |
| EE | q.s. |

Der Wirkstoff, die Maisstärke und der Milchzucker werden unter Zuhilfenahme von EE zu einer homogenen pastösen Mischung verarbeitet. Die Paste wird zerkleinert und das entstehende Granulat wird auf ein geeignetes Blech gebracht und zur Entfernung des Lösungsmittels bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. Verbindungen der allgemeinen Formel I,

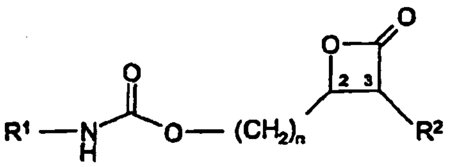

I

worin,

$R^1$ $C_{1-18}$Alkyl, dessen Alkylketten-Kohlenstoffatome gegebenenfalls 1-2-fach durch Sauerstoff und/oder Schwefel ersetzt sind; Phenyl-$C_{0-18}$-alkyl, dessen Phenylgrup- pe gegebenenfalls 1-2-fach durch Halogen, Trifluormethyl, Nitro, $C_{1-8}$-Alkyl, $C_{1-8}$- Alkoxy, $C_{1-6}$-Alkyfearbonyl, $C_{1-6}$-Alkoxycarbonyl, Phenyl, Benzyl und/oder Phenoxy substituiert ist und dessen Alkylketten-Kohlenstoffatome gegebenenfalls 1-2-fach durch Sauerstoff und/oder Schwefel ersetzt sind; $C_{3-7}$Cycloalkyl-$C_{0-18}$-Alkyl, dessen Alkylketten-Kohlenstoffatome gegebenenfalls 1-2-fach durch Sauerstoff und/oder Schwefel ersetzt sind, oder Benzoyl bedeutet;
$R^2$ $C_{1-12}$-Alkyl, dessen Alkylketten-Kohlenstoffatome gegebenenfalls 1-2-fach durch Sauerstoff und/oder Schwefel ersetzt sind; Phenyl-$C_{1-18}$-alkyl, dessen Phenylgrup- pe gegebenenfalls 1-2-fach durch Halogen, $C_{1-4}$-Alkyl, $C_{1-4}$-Alkoxy, Benzyl und/oder Phenoxy substituiert ist und dessen Alkylketten-Kohlenstoffatome gegebenenfalls 1-2-fach durch Sauerstoff und/oder Schwefel ersetzt sind; oder $C_{3-7}$-Cycloalkyl-$C_{0-18}$- Alkyl, dessen Alkylketten-Kohlenstoffatome gegebenenfalls 1-2-fach durch Sau- erstoff und/oder Schwefel ersetzt sind, bedeutet, und

n eine ganze Zahl von 2 bis 8 bedeutet.

**2.** Verbindungen der Formel I gemäß Anspruch 1, worin $R^1$ Phenyl-$C_{0-2}$-alkyl, dessen Phenylgruppe gegebenenfalls durch $C_{1-4}$-Alkyl, $C_{1-4}$-Alkoxy oder Phenoxy substituiert ist, bedeutet.

**3.** Verbindungen der Formel I gemäß Anspruch 1, worin $R^2$ $C_{2-6}$-Alkyl, dessen Alkylketten-Kohlenstoffatome gegebenenfalls 1-2-fach durch Sauerstoff und/oder Schwefel ersetzt sind, bedeutet.

**4.** Verbindungen der Formel I gemäß Anspruch 1, worin n eine ganze Zahl von 2 bis 5 bedeutet.

**5.** Verbindungen der Formel I mach einem der vorstehenden Ansprüche, welche ausgewählt sind aus der Gruppe bestehend aus
5-(3-Hexyl-4-oxo-oxetan-2-yl)-pentyl-(phenoxyphenyl)-carbamat und
4-(3-Hexyl-4-oxo-oxetan-2-yl)-butyl-(phenoxyphenyl)-carbamat.

**6.** Verbindungen der Formel I nach einem der vorstehenden Ansprüche, worin die Substituenten des Kohlenstoffs in 2-Stellung des Lactonrings und die Substituenten des Kohlenstoffs In 3-Stellung des Lactonrings die trans-Stellung zueinander einnehmen.

**7.** Arzneimittel, enthaltend eine pharmakologisch wirksame Menge einer Verbindung der Formel I gemäß Anspruch 1 und übliche pharmazeutische Hilfs- und/oder Trägerstoffe.

**8.** Verwendung von Verbindungen der Formel I gemäß Anspruch 1 für die Herstellung von Arzneimitteln zur Behandlung und/oder Prophylaxe von Obesitas sowie deren Begleit- und/oder Folgeerkrankungen, welche ausgewählt sind aus der Gruppe bestehend aus dem Metabolischen Syndrom, welches Insbesondere Bluthochdruck; Insulinresistenz, vor allem Diabetes mellitus Typ II; Dyslipoproteinämie und Hyperurikämie umfaßt; und kardiovaskulären Erkrankungen, welche Insbesondere koronare Herzkrankheit, cerebrovaskuläre Erkrankungen und die periphere arterielle Verschlußkrankheit umfassen.

**9.** Verfahren zur Herstellung von Verbindungen der allgemeinen Formel I,

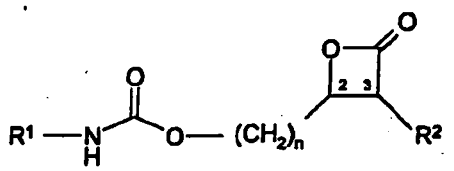

I

worin,

$R^1$ $C_{1-18}$-Alkyl, dessen Alkylketten-Kohlenstoffatome gegebenenfalls 1-2-fach durch Sauerstoff und/oder Schwefel ersetzt sind; Phenyl-$C_{0-18}$-alkyl, dessen Phenylgrup- pe gegebenenfalls 1-2-fach durch Halogen, Trifluormethyl, Nitro, $C_{1-8}$Alkyl, $C_{1-8}$- Alkoxy, $C_{1-6}$-Alkylcarbonyl, $C_{1-6}$-Alkoxycarbonyl, Phenyl, Benzyl und/oder Phenoxy substituiert ist und dessen Alkylketten-Kohlenstoffatome gegebenenfalls 1-2-fach durch Sauerstoff und/oder Schwefel ersetzt sind; $C_{3-7}$-Cycloalkyl-$C_{0-18}$-Alkyl, dessen Alkylkatten-Kohlenstoffatome gegebenenfalls 1-2-fach durch Sauerstoff und/oder Schwefel ersetzt sind oder Benzoyl bedeutet;
$R^2$ $C_{1-12}$-Alkyl, dessen Alkylketten-Kohlenstoffatome gegebenenfalls 1-2-fach durch Sauerstoff und/oder Schwefel ersetzt sind; Phenyl-$C_{1-18}$-alkyl, dessen Phenylgrup- pe gegebenenfalls 1-2-fach durch Halogen, $C_{1-4}$-Alkyl, $C_{1-4}$-Alkoxy, Benzyl und/oder Phenoxy substituiert ist und dessen Alkylketten-Kohlenstoffatome gegebenenfalls 1-2-fach durch Sauerstoff und/oder Schwefel ersetzt sind; oder $C_{3-7}$-Cydoalkyl-$C_{0-18}$- Alkyl, dessen Alkylketten-Kohlenstoffatome gegebenenfalls 1-2-fach durch Sau- erstoff und/oder Schwefel ersetzt sind, bedeutet, und
n eine ganze Zahl von 2 bis 8 bedeutet.

**dadurch gekennzeichnet, daß** man eine Verbindung der allgemeinen Formel II,

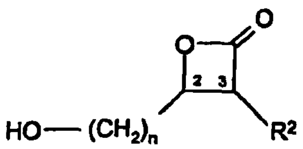

II

worin $R^2$ und n obige Bedeutungen besitzen, mit einer Verbindungder allgemeinen Formel III,

$R^1$-N= C=O III

worin $R^1$ obige Bedeutung besitzt, umsetzt.

**10.** 3-Hexyl-4-{](tetrahydro-2*H*-pyran-2-yl)oxy]pentyl}oxetan-2-on. zur Verwendung als Arzneimittel.

**Claims**

**1.** Compounds of the general formula I,

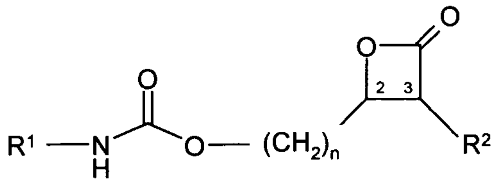

I

wherein

$R^1$ is $C_{1-18}$-alkyl, the alkyl-chain carbon atoms of which are optionally replaced 1-2 times by oxygen and/or sulphur; phenyl-$C_{0-18}$-alkyl, the phenyl group of which is optionally substituted 1-2 times by halogen, trifluoromethyl, nitro, $C_{1-8}$-alkyl, $C_{1-8}$- alkoxy, $C_{1-6}$-alkylcarbonyl, $C_{1-6}$-alkoxycarbonyl, phenyl, benzyl and/or phenoxy and the alkyl-chain carbon atoms of which are optionally replaced 1-2 times by oxygen and/or sulphur; $C_{3-7}$-cycloalkyl-$C_{0-18}$-alkyl, the alkyl-chain carbon atoms of which are optionally replaced 1-2 times by oxygen and/or sulphur, or benzoyl;
$R^2$ is $C_{1-12}$-alkyl, the alkyl-chain carbon atoms of which are optionally replaced 1-2 times by oxygen and/or sulphur; phenyl-$C_{1-18}$-alkyl, the phenyl group of which is optionally substituted 1-2 times by halogen, $C_{1-4}$-alkyl, $C_{1-4}$-alkoxy, benzyl and/or phenoxy and the alkyl-chain carbon atoms of which are optionally replaced 1-2 times by oxygen and/or sulphur; or $C_{3-7}$-cydoalkyl-$C_{0-8}$-alkyl, the alkyl-chain carbon atoms of which are optionally replaced 1-2 times by oxygen and/or sulphur, and
n is a whole number from 2 to 8.

**2.** Compounds of Formula I according to Claim 1, wherein $R^1$ is phenyl-$C_{0-2}$-alkyl, the phenyl group of which is optionally substituted by $C_{1-4}$-alkyl, $C_{1-4}$-alkoxy or phenoxy.

**3.** Compounds of Formula I according to Claim 1, wherein $R^2$ is $C_{2-6}$-alkyl, the alkyl-chain carbon atoms of which are optionally replaced 1-2 times by oxygen and/or sulphur.

**4.** Compounds of Formula I according to Claim 1, wherein n is a whole number from 2 to 5.

**5.** Compounds of Formula I according to one of the preceding claims, which are selected from the group consisting of
5-(3-hexyl-4-oxo-oxetan-2-yl)-pentyl-(phenoxyphenyl)-carbamate and
4-(3-hexyl-4-oxo-oxetan-2-yl)-butyl-(phenoxyphenyl)-carbamate.

**6.** Compounds of Formula I according to one of the preceding claims, wherein the substituents of the carbon in the 2 position of the lactone ring and the substituents of the carbon in the 3 position of the lactone ring adopt the trans

position relative to one another.

7. Medicament, containing a pharmacologically effective quantity of a compound of Formula I according to Claim 1 and conventional pharmaceutical auxiliaries and/or excipients.

8. The use of compounds of Formula I according to Claim 1 for the preparation of medicaments for the treatment and/or prophylaxis of obesity and its accompanying and/or concomitant diseases, which are selected from the group consisting of metabolic syndrome, which comprises in particular hypotension; insulin resistance, above all Type II diabetes mellitus; dyslipoproteinaemia and hyperuricaemia; and cardiovascular diseases, which comprise in particular coronary heart disease, cerebrovascular diseases and peripheral occlusive arterial disease.

9. A process for the preparation of compounds of the general formula I,

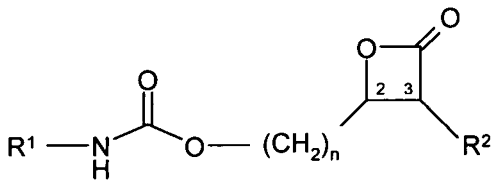

I

wherein

$R^1$ is $C_{1-18}$-alkyl, the alkyl-chain carbon atoms of which are optionally replaced 1-2 times by oxygen and/or sulphur; phenyl-$C_{0-8}$-alkyl, the phenyl group of which is optionally substituted 1-2 times by halogen, trifluoromethyl, nitro, $C_{1-8}$-alkyl, $C_{1-8}$- alkoxy, $C_{1-6}$-alkylcarbonyl, $C_{1-6}$-alkoxyearbonyl, phenyl, benzyl and/or phenoxy and the alkyl-chain carbon atoms of which are optionally replaced 1-2 times by oxygen and/or sulphur; $C_{3-7}$-cycloalkyl-$C_{0-18}$-alkyl, the alkyl-chain carbon atoms of which are optionally replaced 1-2 times by oxygen and/or sulphur, or benzoyl;
$R^2$ is $C_{1-12}$-alkyl, the alkyl-chain carbon atoms of which are optionally replaced 1-2 times by oxygen and/or sulphur; phenyl-$C_{1-18}$-alkyl, the phenyl group of which is optionally substituted 1-2 times by halogen, $C_{1-4}$-alkyl, $C_{1-4}$-alkoxy, benzyl and/or phenoxy and the alkyl-chain carbon atoms of which are optionally replaced 1-2 times by oxygen and/or sulphur; or $C_{3-7}$-cycloalkyl-$C_{0-18}$-alkyl, the alkyl-chain carbon atoms of which are optionally replaced 1-2 times by oxygen and/or sulphur, and
n is a whole number from 2 to 8.

**characterised in that** a compound of the general formula II,

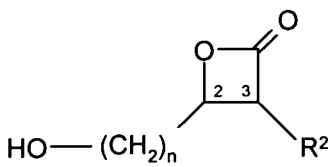

II

wherein $R^2$ and n have the above meanings, is reacted with a compound of the general formula III,

$R^1$-N=C=O III

wherein $R^1$ has the above meaning.

10. 3-Hexyl-4-{5[(tetrahydro-2H-pyran-2-yl)oxy]pentyl}oxetan-2-one for use as medicaments.

**Revendications**

1. Composés de formule générale I,

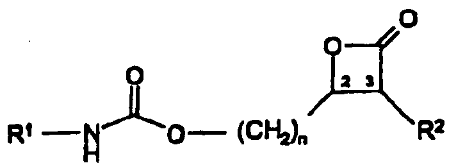

I

où

$R^1$ signifie $C_{1-18}$-alkyle, dont les atomes de carbone de la chaîne alkyle sont le cas échéant remplacés une à deux fois par oxygène et/ou soufre ; phényl-$C_{0-18}$- alkyle, dont le groupe phényle est le cas échéant monosubstitué ou disubstitué par halogène, trifluorométhyle, nitro, $C_{1-8}$-alkyle, $C_{1-8}$-alcoxy, $C_{1-6}$-alkylcarbonyle, $C_{1-6}$-alcoxycarbonyle, phényle, benzyle et/ou phénoxy et dont les atomes de carbone de la chaîne alkyle sont le cas échéant remplacés une à deux fois par oxygène et/ou soufre ; $C_{3-7}$-cycloalkyl-$C_{0.18}$-alkyle, dont les atomes de carbone de la chaîne alkyle sont le cas échéant remplacés une à deux fois par oxygène et/ou soufre, ou benzoyle ;
$R^2$ signifie $C_{1-12}$-alkyle, dont les atomes de carbone de la chaîne alkyle sont le cas échéant remplacés une à deux fois par oxygène et/ou soufre ; phényl-$C_{1-18}$- alkyle, dont le groupe phényle est le cas échéant monosubstitué ou disubstitué par halogène, $C_{1-4}$- alkyle, $C_{1-4}$-alcoxy, benzyle et/ou phénoxy et dont les atomes de carbone de la chaîne alkyle sont le cas échéant remplacés une à deux fois par oxygène et/ou soufre ; ou $C_{3-7}$-cycloalkyl-$C_{0-18}$-alkyle, dont les atomes de carbone de la chaîne alkyle sont le cas échéant remplacés une à deux fois par oxygène et/ou soufre, et
n signifie un nombre entier de 2 à 8.

**2.** Composés de formule I selon la revendication 1, où $R^1$ signifie phényl-$C_{0-2}$-alkyle, dont le groupe phényle est le cas échéant substitué par $C_{1-4}$-alkyle, $C_{1-4}$-alcoxy ou phénoxy.

**3.** Composés de formule I selon la revendication 1, où $R^2$ signifie $C_{2-6}$-alkyle, dont les atomes de carbone de la chaîne alkyle sont le cas échéant remplacés une à deux fois par oxygène et/ou soufre.

**4.** Composés de formule I selon la revendication 1, où n signifie un nombre entier de 2 à 5.

**5.** Composés de formule I selon l'une quelconque des revendications précédentes, qui sont choisis dans le groupe constitué par

- 5-(3-hexyl-4-oxo-oxétan-2-yl)-pentyl-(phénoxyphényl)-carbamate et
- 4-(3-hexyl-4-oxo-oxétan-2-yl)-butyl-(phénoxyphényl)-carbamate.

**6.** Composés de formule I selon l'une quelconque des revendications précédentes, où les substituants du carbone en position 2 du cycle lactone et les substituants du carbone en position 3 du cycle lactone se trouvent dans une position trans l'un par rapport à l'autre.

**7.** Médicament, contenant une quantité pharmacologiquement active d'un composé de formule I selon la revendication 1 et des adjuvants et/ou des substances support pharmaceutiques usuel(le)s.

**8.** Utilisation de composés de formule I selon la revendication 1 pour la préparation de médicaments destinés au traitement et/ou à la prophylaxie de l'obésité ainsi que leurs maladies associées et/ou secondaires, qui sont choisies dans le groupe constitué par le syndrome métabolique, qui comprend en particulier l'hypertension ; la résistance à l'insuline, surtout le diabète sucré de type II ; la dyslipoprotéinémie et l'hyperuricémie ; et les maladies cardiovasculaires, qui comprennent en particulier la maladie coronarienne, les maladies cérébrovasculaires et l'endartérite oblitérante.

**9.** Procédé pour la préparation de composés de formule générale I,

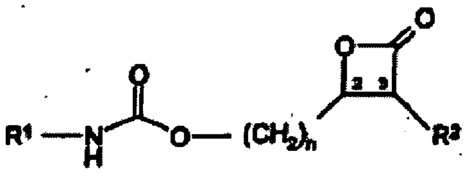

I

où

$R^1$ signifie $C_{1-18}$-alkyle, dont les atomes de carbone de la chaîne alkyle sont le cas échéant remplacés une à deux fois par oxygène et/ou soufre ; phényl-$C_{0-18}$- alkyle, dont le groupe phényle est le cas échéant monosubstitué ou disubstitué par halogène, trifluorométhyle, nitro, $C_{1-8}$-alkyle, $C_{1-8}$-alcoxy, $C_{1-6}$-alkylcarbonyle, $C_{1-6}$-alcoxycarbonyle, phényle, benzyle et/ou phénoxy et dont les atomes de carbone de la chaîne alkyle sont le cas échéant remplacés une à deux fois par oxygène et/ou soufre ; $C_{3-7}$-cycloalkyl-$C_{0-18}$-alkyle, dont les atomes de carbone de la chaîne alkyle sont le cas échéant remplacés une à deux fois par oxygène et/ou soufre, ou benzoyle ;
$R^2$ signifie $C_{1-12}$-alkyle, dont les atomes de carbone de la chaîne alkyle sont le cas échéant remplacés une à deux fois par oxygène et/ou soufre ; phényl-$C_{1-18}$- alkyle, dont le groupe phényle est le cas échéant monosubstitué ou disubstitué par halogène, $C_{1-4}$- alkyle, $C_{1-4}$-alcoxy, benzyle et/ou phénoxy et dont les atomes de carbone de la chaîne alkyle sont le cas échéant remplacés une à deux fois par oxygène et/ou soufre ; ou $C_{3-7}$-cycloalkyl-$C_{0-18}$-alkyle, dont les atomes de carbone de la chaîne alkyle sont le cas échéant remplacés une à deux fois par oxygène et/ou soufre, et
n signifie un nombre entier de 2 à 8,

**caractérisé en ce qu'**on transforme un composé de formule générale II

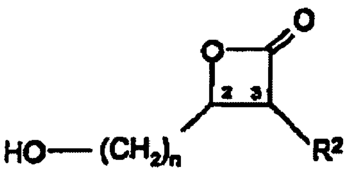

II

où $R^2$ et n présentent les significations ci-dessus, avec un composé de formule générale III,

**$R^1$-N=C=O** III

où $R^1$ présente la signification ci-dessus.

**10.** 3-hexyl-4-{5[(tétrahydro-2H-pyran-2-yl)oxy]pentyl}oxétan-2-one destiné à une utilisation comme médicament.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0444482 A2
- EP 0129748 A1
- WO 03050154 A2


### In der Beschreibung aufgeführte Nicht-Patentliteratur


- **Silvia Cardani ; Carlo De Toma ; Cesare Gennari ; Carlo Scolastico.** Titanium Tetrachloride-Mediated Enantioselective Synthesis of trans β-Lactones. *Tetrahedron,* 1992, vol. 48 (27), 5557-5564
- **Scott G. Nelson ; Cheng Zhu ; Xiaoqiang Shen.** Catalytic Asymmetric Acyl Halide-Aldehyde Cyclocondensation Reactions of Substituted Ketens. *J. Am. Chem. Soc.,* 18. Dezember 2003, vol. 126, 14-15
- **Hong Woon Yang ; Cunxiang Zhao ; Daniel Romo.** Studies of the tandem Mukaiyama aldol-lactonization (TMAL) reaction: A consice and highly diastereoselective route to beta-lactones applied to the total synthesis of the potent pancreatic lipase inhibitor, (-)-Panclicin D. *Tetradehdron,* 01. Dezember 1997, vol. 53 (48), 16471-16488
- **Keith T. Mead ; Hui-Li Yang.** A new approach to the preparation of 2-substituted tetrahydrofurans with alpha-syn selectivity. *Tetrahedron Letters,* 1989, vol. 30 (49), 6829-6832
- **Cunxiang Zhao ; Daniel Romo.** A beta-Lactone-based Route to Cyclopentanes via Intramolecular Allylsilane Additions. An unexpected Friedel-Crafts Alkylation. *Tetrahedron Letters,* 15. September 1997, vol. 38 (37), 6537-6540
- **A. Pommier ; J.-M. Pons.** *Synthesis,* 1993, vol. 5, 441-459
- **McOmie.** Protective Groups in Organic Chemistry. Plenum Press
- **Greene ; Wuts.** Protective Groups in Organic Synthesis. Wiley Interscience Publication
- **H.W. Yang et al.** *Tetrahedron,* 1997, vol. 53 (48), 16471-16488